(19)

**Europäisches Patentamt**
**European Patent Office**
**Office européen des brevets**

(11) **EP 3 973 281 B1**

(12) **EUROPEAN PATENT SPECIFICATION**

(45) Date of publication and mention
of the grant of the patent:
**18.03.2026 Bulletin 2026/12**

(21) Application number: **20726829.3**

(22) Date of filing: **20.05.2020**

(51) International Patent Classification (IPC):
**A61B 5/083** (2006.01)          **G01N 27/404** (2006.01)
**G01N 33/497** (2006.01)

(52) Cooperative Patent Classification (CPC):
**G01N 27/4045; A61B 5/083; G01N 33/497;**
**G01N 33/4975**

(86) International application number:
**PCT/EP2020/064041**

(87) International publication number:
**WO 2020/234338 (26.11.2020 Gazette 2020/48)**

(54) **DISPOSABLE WEARABLE SENSOR FOR CONTINUOUS MONITORING OF BREATH BIOCHEMISTRY**

TRAGBARER EINWEGSENSOR ZUR KONTINUIERLICHEN ÜBERWACHUNG DER ATEMBIOCHEMIE

CAPTEUR PORTABLE JETABLE POUR LA SURVEILLANCE CONTINUE DE BIOCHIMIE RESPIRATOIRE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **20.05.2019 DE 102019113253**
**30.08.2019 EP 19194595**

(43) Date of publication of application:
**30.03.2022 Bulletin 2022/13**

(73) Proprietors:
• **Albert-Ludwigs-Universität Freiburg**
**79098 Freiburg (DE)**
• **Imperial College London**
**London SW7 2AZ (GB)**

(72) Inventors:
• **DINCER, Can**
**79115 Freiburg (DE)**
• **LAUBENDER, Elmar**
**79106 Freiburg (DE)**
• **MAIER, Daniela**
**59073 Hamm (DE)**
• **SCHUMANN, Stefan**
**79114 Freiburg (DE)**

• **URBAN, Gerald**
**79104 Freiburg (DE)**
• **GUDER, Firat**
**London Greater London SW11 5PN (GB)**

(74) Representative: **Hertin und Partner**
**Rechts- und Patentanwälte PartG mbB**
**Kurfürstendamm 63**
**10707 Berlin (DE)**

(56) References cited:
**WO-A1-2010/045458      WO-A1-2010/045458**
**WO-A1-2012/067511      WO-A1-2012/067511**
**WO-A1-2013/090999      WO-A1-2013/090999**
**WO-A1-2015/054775      WO-A1-2015/054775**
**WO-A1-98/44342         WO-A1-98/44342**
**US-A1- 2013 091 924    US-A1- 2013 091 924**

• **GHOREISHIZADEH SARA S ET AL: "An integrated platform for differential electrochemical and ISFET sensing", 2016 IEEE INTERNATIONAL SYMPOSIUM ON CIRCUITS AND SYSTEMS (ISCAS), IEEE, 22 May 2016 (2016-05-22), pages 2875 - 2878, XP032942199, DOI: 10.1109/ISCAS.2016.7539193**

**(Cont. next page)**

- **FIRAT G�DER ET AL: "Paper-Based Electrical Respiration Sensor", ANGEWANDTE CHEMIE, INTERNATIONAL EDITION, vol. 55, no. 19, 4 May 2016 (2016-05-04), DE, pages 5727 - 5732, XP055668627, ISSN: 1433-7851, DOI: 10.1002/anie.201511805**
- **GHOREISHIZADEH SARA S ET AL: "An integrated platform for differential electrochemical and ISFET sensing", 2016 IEEE INTERNATIONAL SYMPOSIUM ON CIRCUITS AND SYSTEMS (ISCAS), IEEE, 22 May 2016 (2016-05-22), pages 2875 - 2878, XP032942199, DOI: 10.1109/ISCAS.2016.7539193**

- **FIRAT GÜDER ET AL: "Paper-Based Electrical Respiration Sensor", ANGEWANDTE CHEMIE, INTERNATIONAL EDITION, vol. 55, no. 19, 4 May 2016 (2016-05-04), DE, pages 5727 - 5732, XP055668627, ISSN: 1433-7851, DOI: 10.1002/anie.201511805**

## Description

The invention relates to an electrochemical method and an electrochemical sensor for breath analysis of single or multiple analytes using a porous, preferably flexible and disposable supporting material, wherein a salt is incorporated, and which can be wetted in contact with the exhaled breath, acting simultaneously as sample collection (sampling) method, as an electrolyte and as a support for the electrode structures. The salt is hygroscopic, such that the porous substrate stays wet.

To ensure that the obtained signal originates from the analyte, the electrochemical sensor exhibits a differential electrode design, comprising a sensing (analyte-sensitive) and a blank (analyte-insensitive) electrode in order to isolate and remove the background signals. In a preferred embodiment, the sensing electrode comprises different mediators, like Prussian Blue or Cobalt Phthalocyanine, or can be out of or modified with Platinum or Gold to allow for the detection of hydrogen peroxide as an analyte. The approach can however be simply adapted for a wide range of substances by changing or modifying and/or coating the material of the sensing electrode and/or the supporting material. The sensor is further compatible with existing medical equipment and thus, can be employed in a variety of applications, like wearable, on-site or clinical monitoring of exhaled breath.

## BACKGROUND OF THE INVENTION

The global air pollution has been rising at an alarming rate due to the advancing industrialization and dependency on motorized vehicles. This leads increasingly to severe health problems'. For example, disorders concerning lung and airways, such as asthma, lung cancer and chronic obstructive pulmonary disease, are on the rise according to World Health Organization[2]. 3.9 million deaths each year worldwide are caused by respiratory diseases[3,4]. A large proportion of respiratory diseases are chronic and require frequent check-ups to monitor their progression. Inflammatory cells, such as macrophages and neutrophils, produce hydrogen peroxide ($H_2O_2$) in reaction to respiratory diseases[5,6]. Detection of $H_2O_2$ in exhaled breath or other biomarkers of respiratory illnesses may provide a non-invasive route to detect these diseases quickly, non-invasively and inexpensively.

Unfortunately, $H_2O_2$ is difficult to measure in exhaled breath since it is easily oxidized in air and is not stable with increasing pH in aqueous solutions, including exhaled breath condensate (EBC) with a pH between 7.8 to 8.1[7-9].

For analysis of exhaled breath, EBC is first collected and then measured in centralized laboratories[10,11]. The sample collection is done by cooling the exhaled breath of a patient and breathing into a special device that consists of a mouthpiece and a cooled tube. The EBC is analysed where the $H_2O_2$ concentration is determined using electrochemical (amperometric) or optical (fluorometric, colorimetric, chemiluminescence or fluorescence) methods of detection[6,12]. Devices for the EBC collection and analysis are already commercially available (ECoScreen® and ECoCheck® by FILT, Germany), however, due to their size and cost, they are not suitable for an on-site or wearable continuous monitoring. Furthermore, the current approaches for analysing EBC are susceptible to errors since the sample collected is influenced by the duration of storage, temperature, amount of saliva, changing flow and volume of exhaled air[13].

A further approach using exhaled breath is described in WO 2012/067511 A1, which refers to a device for the analysis of exhaled air, e.g. with respect to hydrogen peroxide. The proposed sensor comprises a membrane which is applied to a glass-based chip and is intended to absorb hydrogen peroxide. Serving as a support on the glass-based chip a working electrode (WE), counter electrode (CE) and reference electrode (RE) are applied and configured for measuring the concentration of hydrogen peroxide. The process is supported by a Peltier element. A salt or electrolyte solution may be preferably introduced into the membrane adjacent to the electrodes. Due to using a solid glass-based chip as a support for the electrodes the sensor itself is not air permeable and thus cannot be easily implemented as a wearable sensor. Instead the sensor is implemented into a more complex device including a conventional exhaled breath supply and conditioning unit. Furthermore, the design of the electrodes in WO 2012/067511 A1 lacks the possibility for a background correction.

US 2013/00919224 A1 relates to system for a simple functional test ('bump check') of a gas sensor. The proposed sensor comprises two electrochemical sensors in the same sensor housing. A first working electrode is configured for the gas to be detected, while a second working electrode enables the detection of breathing air. The functionality of the gas sensor (including the permeability of an inlet) can be assessed by simply breathing into the sensor. The two working electrodes and are connected to each other via a matrix saturated with electrolytes (electrolyte saturate wick material) and to a counter electrode and reference electrode. A method for monitoring or detecting analytes in an exhaled air based on a differential measurement using both working electrodes is not disclosed.

Ghoreishizadeh Sara S. et al. disclose a fully integrated differential biosensing platform on CMOS (Complementary metal-oxide-semiconductor) for miniaturized enzyme-based electrochemical sensing[33].

Güder et al. disclose a paper-based electrical respiration sensor. The sensor is a paper-based moisture sensor that uses the hygroscopic character of paper to measure patterns and rate of respiration by converting the changes in humidity caused by the cycles of inhalation and exhalation to electrical signals[34].

WO 98/44342 relates to a measuring device which comprises electrodes fabricated on porous membrane substrate in which the sample migrates chromatographically; and a method for quantifying material in the sample by using the device.

WO 2013/090999 A1 relates to devices for respiratory support, e.g., in cases of sleep apnea syndrome (SAS) and proposes integrating a sensor module into such devices for analyzing exhalation. The sensor module includes, for example, a collector for collecting EBC (exhaled breath condensate).

Wearable sensors may provide a viable alternative to monitor constituents of exhaled breath, without reliance on EBC, for accelerated and inexpensive diagnosis of respiratory diseases. Wearable devices already exist in various forms including smart watches, fitness bracelets or digital glasses[14-18]. The concept of wearable sensors has already been accepted by the general public and their adoption has increased dramatically in the past few years[14]. For biochemical analysis, however, reusability of a medical device is often out of the question, therefore, a wearable sensor for monitoring exhaled breath must be low-cost and disposable.

For biochemical analysis, paper as substrate offers many advantages: it is (i) easy-to-handle, (ii) permeable for gases, (iii) capable of absorbing fluids and allow their passive transport, (iv) can be folded, (v) has a good compatibility with chemicals and biomolecules, (vi) is cost-effective, (vii) hygroscopic and (viii) disposable[19-21]. Furthermore, the cellulose fibres can easily be functionalized to modify the paper's permeability, hydrophilicity or reactivity, by patterning with wax, inks, polymers or biomolecules (such as enzymes)[22].

A notable example for paper based wearable devices is the electrical respiration sensor introduced by Güder and colleagues[23]. In this approach, the respiratory activity (such as breathing rate, relative volume etc.) of person can be observed using a wearable mask with an integrated paper based sensing device. This sensor comprises two printed carbon electrodes on an ordinary cellulose paper. The detecting principle is electrical and based on the change in moisture in the paper. This again results in a detectable change of the conductivity and thus, obtained signal between the carbon electrodes while breathing. The cellulose paper is able to absorb up to 10% of its own weight in water from humidity in air. This device can be used without calibration since only the changes between inhaled and exhaled air are measured. It is suitable for on-site monitoring as it is simple to use via a mobile app and does not require any additional equipment. This device, however, is limited to the determination of the physical quantities related to breathing and cannot provide any biochemical information that may be extracted from exhaled breath.

Komkova et al.[26] describes a hydrogen peroxide detection in wet air flow (i.e. only for continuous exhaling) with a commercial Prussian Blue based three electrode systems. The electrodes are assembled on a solid support planar system, wherein a separate filament membrane made of cotton textile is used for bridging the electrodes. The sensitivity of the device, however, does not allow for a direct analysis of the breath, but requires the provision of highly dispersed aerosol, i.e. wet air and intermediate steps for processing. Furthermore, the manufacturing of the device is complicated by the assembly of multiple components, which also increase production costs. In this regard, variations in the contacting of the two components can lead to measurement errors.

A need for improvement to provide alternative additional means or methods for an electrochemical breath analysis e.g. in relation to hydrogen peroxide to allow for a non-invasive monitoring of respiratory diseases exist.

A further medical field which may profit from such improved additional means or methods for an electrochemical analysis of breath is Diabetes.

Diabetes is a chronic, metabolic disease characterized by elevated levels of blood glucose (BG), which leads over time to serious injuries of various organs such as the heart, blood vessels, eyes, kidneys and nerves. According to World Health Organization (WHO), about 422 million people worldwide have diabetes, particularly in low-and middle-income countries, and 1.6 million deaths are directly attributed to diabetes each year[26].

Living a long and healthy life for people with diabetes is only possible by control of the BG concentration in the recommended range of 70 - 180 mg/dl (3.9 - 10.0 mmol/L). Herein, the most effective method to achieve tight control is to self-monitor the blood glucose levels as frequent as possible, preferably in a continuous manner. Over decades, finger prick blood sugar tests using a disposable test strip along with a non-disposable glucose meter have become the most common and widely available method thanks to its reasonable accuracy and precision, convenience, and its ability to provide real-time plasma glucose levels on demand[27,28].

Despite the recent advantages in the self-monitoring of blood glucose, glycemic control is still a challenge for many diabetes patients, especially for those with type II diabetes. As a number of factors discourage the use of a finger-prick method, especially the pain due to repeated finger pricks with lancets to draw blood sample, fear of infection during the pricking process, cost of test accessories, and the invasiveness to their daily life. Another shortcoming of the finger prick tests is the ability to only provide a discrete snapshot of the BG levels. While the blood glucose concentration changes continuously during the day, a patient might miss important hyperglycemic and hypoglycemic events between the discrete finger prick tests[27].

In this sense, monitoring the BG level continuously would enable a better regulation of these glycemic episodes and thus, avoiding physiological complications[29]. To achieve continuous glucose monitoring (CGM) several methods have been developed[27,30]. The most common method is to have a flexible needle placed under the skin with a data transmitting device sitting on the skin, which continuously monitors interstitial fluid (ISF) glucose levels. Still, the flexible subcutaneous needle must be inserted into the skin by an invasive guide needle and therefore, same disadvantages still exist regarding the invasiveness of the technique, including the pain and fear of infection and the replacement of the sensor every few weeks. Therefore, the demand and expectation for a truly non-invasive blood glucose monitor is growing higher and higher[31].

Non-invasive sensing methods could have the ability to overcome the issues with the discourage of diabetes patients since they provide more comfortable and still clean way to monitor the blood glucose concentration[30,31]. However, practical solutions allowing for a straightforward implementation of a sensor for blood glucose concentration based on human breath, implemented in a wearable have not been described.

In light of the prior art, there remains a significant need to provide additional means or methods for an electrochemical breath analysis of single or multiple analytes.

## SUMMARY OF THE INVENTION

An objective of the invention is to provide a sensor and a method that overcomes the disadvantages of the state-of-the-art in providing alternative and/or improved means for monitoring analytes in the breath of a subject. In particular, this invention aims at realizing an electrochemical sensor and a method of using the sensor for breath analysis, in particular for point-of-care applications, that is compact, low-cost, wearable and allows for a continuous monitoring of analytes directly from the exhaled breath.

The objective is solved by the features of the independent claims. Preferred embodiments of the present invention are provided by the dependent claims.

In a first aspect the invention relates to an electrochemical sensor for monitoring the presence of an analyte in the breath of a subject, comprising

  i. a support comprising a porous substrate material
  ii. at least one pair of working electrodes as well as at least one counter and/or reference electrode, which are at least partially integrated into said porous supporting material

  characterized in that the at least two working electrodes comprise an analyte-sensitive sensing electrode and an analyte-insensitive blank electrode and wherein a salt is immobilized in said support, such that upon exhaling onto the sensor a differential electrochemical measurement at said pair of working electrodes allows for monitoring the presence of the analyte in the breath of said subject and
  wherein the support is air-permeable such that the breath may at least partially flow through the electrochemical sensor and hygroscopic such that a liquid portion of the breath is captured to allow for dissolution of the salt immobilizes in said support and
  wherein the salt immobilized in said support is hydrophilic to an extent that the humidity of human exhaled breath is sufficient to form a conductive electrolyte, and the salt immobilized in said support is hygroscopic to an extent sufficient to keep the porous substrate material wet.

The invention provides an electrochemical sensor and an electrochemical differential method for continuous on-site breath testing using flexible, porous and disposable supporting materials for the electrolyte and the electrodes, which overcomes current bottlenecks by allowing a direct and long-term on-site monitoring in a rapid, facile and inexpensive way. Thanks to its ability to measure directly the exhaled breath (without any sample collection of exhaled breath condensate), the approach is easier to implement and less error-prone. In particular, the salt is immobilized in the porous substrate material, such that upon exhaling onto the sensor a differential electrochemical measurement at said pair of working electrodes allows for monitoring the presence of the analyte in the breath of the subject. External or further supply of a liquid is not necessary. Instead, the breath itself provides the necessary humidity. Herein, the salt immobilized in said porous support dissolves in the liquid portion of the breath to form an electrolyte allowing for an electrochemical measurement at the two pairs of working electrodes. In this regard, a particular advantage of the sensor is the provision of a sensing and a blank electrode. The difference of the electrochemical signals provided by the two electrodes allows for a particularly confident readout of the presence and/or concentration of an analyte in the breath. While the sensing electrode specifically reports on the presence of the analyte, the blank electrode allows for removing of the non-specific signal by a mathematical operation.

The sensor and method are characterized by a number of distinguishing features. First, to guarantee that the measured signal originates from the target substance, the sensor has a differential electrode design allowing for differential electrochemical measurement at said pair of working electrodes. Herein, one of the working electrodes is an analyte-sensitive sensing electrode, while the other electrode is an analyte-insensitive blank electrode. Whereas analyte-sensitive sensing electrode reliably reports on the presence of the analyte in the breath, the analyte-insensitive blank electrode allows for monitoring background signals that do not stem from the presence of analyte. By means of the differential electrode design, the background signals occurring due to interfering substances and/or environmental conditions (temperature, humidity etc.), as well as the periodic fluctuations caused by the respiratory action can be subtracted from the sensor signals and corrected in real time. Second, by changing or modifying and/or coating the material of substrate or the sensing electrode (for instance, with metal, metal oxide- or semiconducting micro- and nanoparticles, enzymes, selective membranes or conducting polymers), the presented differential approach can be easily extended for a large number of analytes. Third, a flexible and porous support, such as paper, has the additional advantage that it can be shaped and patterned in a way that the sensing surface as well as the collection volume can be considerably increased. Fourth, the orientation and porosity of the sensing surface can be tuned to minimize breathing resistance and to improve signal quality (i.e. signal-to-noise ratio).

The invention thus preferably combines the unique properties (i.e. lightweight, hygroscopic, capable of absorbing fluids and acting like a solid electrolyte) of cellulose-based materials such as paper along with a differential electrochemical detection to sample and analyze respiratory fluid directly from exhaled breath. This allows for a completely non-invasive and disposable wearable approach to directly and continuously measure a number of different analytes including blood glucose levels or hydrogen peroxide in a particular simple and low-cost manner.

In a preferred embodiment of the invention the support is flexible. The flexibility of the support allows for an easy implementation of the electrochemical sensor into different breath analysis and/or monitoring systems. Furthermore, being air-permeable the breath may at least partially flow through the electrochemical sensor. Air permeability may be quantified as a rate of airflow (e.g. breath) passing perpendicularly through an area of support. The air permeability may depend on the thickness of the support, which may for instance be less than 5 mm, preferably less than 4 mm, 3 mm, 2 mm or 1 mm. Support materials such as cellulose provide particular good air permeability, which ensures that a long-term observation of the breath can be conducted, while comfortably breathing. Hygroscopic preferably means that the material of the support is designed to absorb moisture from the air or breath. The preferred porous materials as described herein ensure a hygroscopic support that may thus capture the liquid portion of the breath to allow for dissolution of the immobilized salt and precise electrochemical measurements as described herein.

Contrary to solid supports, which are not air-permeable (such as a glass-based support described in WO 2012/067511 A1) the preferred embodiment allows for a straight-forward implementation of the entire sensor as a wearable system. Herein, the electrodes are at least partially integrated into an air-permeable support, such that a breathing through the sensor itself is possible. Using a hygroscopic support furthermore assists the absorption of moisture from the breath, such that the breath humidity dissolves the salt immobilized in the porous support and provides for electrolytes allowing for electrochemical measurements as described herein.

In some embodiments additional slits or openings may be provided within the support to facilitate the breathing. In this case not the entire exhaled breath flows through the porous material, but some of the air is allowed to flow through said slits. Said embodiment further lowers the breathing resistance and augments the wearing comfort for the user. Advantageously, since a substantial part of the breath still flows through the substrate the detection quality is not compromised.

In a preferred embodiment of the invention the support is selected from a group consisting of porous membranes, cellulose-based materials (like paper), a ceramic, a hydrogel and/or a hydrophilic polymer. These materials are particularly suited to provide the desired properties of a flexible, hygroscopic and/or air-permeable support.

The electrochemical sensor is characterized in that the pair of working electrodes and the at least one reference or counter electrode are at least partly integrated into the porous support.

Partly integrated preferably means that at least 5%, 10%, 20%, 30%, 40%, 50%, more preferably at least 60%, 70%, 80%, 90% or even 100% of the material that the electrodes are made of is situated within the porous material of the support. To this end part of material of the electrodes may for instance be brought into the support by filling the gaps and/or voids of the porous material. In a particular preferred embodiment, a conductive paste, e.g. carbon or metal pastes, may be applied to submerge with the porous substrate.

Such integration enhances the detection efficiency as electrodes, the salt/electrolyte and analyte are interacting within the support itself, providing for high reaction volumes.

In a further preferred embodiment of the invention the electrochemical sensor is characterized in that the analyte is selected from the group consisting of hydrogen peroxide, glucose, lactate pathogens, genetic materials, antibiotics, protein-based biomarkers, hormones, hydrocarbons, aldehydes, sulfides, ammonia, ethanol, acetone, isoprene, ethane, carbonyl sulfides, carbon dioxides, carbon monoxide, nitrogen monoxide and volatile organic compounds (VOCs).

An advantage of the sensor and method described herein, is its flexible use for a number of analytes including but not limited to the once mentioned above. In order to allow for a detection of the analyte, an analyte-sensitive material may be chosen which acts as a catalyst or mediator such that an electrochemical reaction is supported in the presence of an analyte resulting in a detectable signal at the working electrodes.

In a further preferred embodiment of the invention the electrochemical sensor is characterized in that the sensing electrode comprise an analyte-sensitive material, which is a catalyst for an electrochemical reaction of the analyte. In some embodiments the analyte-sensitive material (such as enzymes) may also be positioned within the porous substrate, but in the close vicinity of the sensing electrodes, such that an analyte-dependent signal is specifically generated at the sensing, but not at the blank electrode, which can be separated by a diffusion barrier.

In a further preferred embodiment, the sensing electrode comprises an analyte-sensitive receptor (for example, an antibody), which causes an impedance signal change in dependence of the analyte concentration.

In a further preferred embodiment of the invention the electrochemical sensor is characterized in that the material of the sensing electrode is supplemented with and/or coated with an analyte-sensitive material, preferably selected from the group consisting of metal, metal oxide or semiconducting micro- or nanoparticles, enzymes, selective membranes and conductive polymers.

In a preferred embodiment the analyte is glucose and the sensing electrode comprises a catalyst for glucose, e.g. glucose oxidase, as the analyte-sensitive material.

To this end in a preferred embodiment glucose oxidase may be immobilized in a compartment surrounding the sensing electrode, e.g. by solving glucose oxidase in a buffer solution and allowing for an adsorption into a compartment of the porous substrate. Further preferred embodiments may refer to a covalent immobilization of glucose oxidase (for example, by using glutaraldehyde), an encapsulation (e.g. by polyethyleneimine), or entrapment in a gel (such as a hydrogel) into a porous substrate. To detect the presence of glucose, the glucose oxidase may catalyse the oxidation of glucose into hydrogen peroxide ($H_2O_2$) which can be reduced for instance at a screen-printed Prussian Blue (PB)-mediated or Cobalt Phthalocyanine-mediated carbon electrode. Measurement of the current at the sensing electrode will thus directly report on glucose concentration of the sample.

In a preferred embodiment the blank analyte-insensitive electrode may be situated in a compartment of the porous substrate, in which no analyte-sensitive material, such as glucose oxidase, is present. Therefore, the signal detected at said blank electrode may advantageously be used for a differential measurement as described herein.

In a preferred embodiment the analyte is lactate and the sensing electrode comprises as a catalyst for lactate, e.g. lactate oxidase, as the analyte-sensitive material.

In a preferred embodiment the analyte is hydrogen peroxide and the sensing electrode comprises a mediator, e.g. Prussian Blue or Cobalt Phthalocyanine, as the analyte-sensitive material.

As detailed above a large proportion of respiratory diseases are accompanied by an augmented production of $H_2O_2$, detectable in an exhaled breath may provide a non-invasive route to detect these diseases. Prussian Blue deposited on the electrode surface under certain conditions has been described to be a selective electrocatalyst of hydrogen peroxide ($H_2O_2$) reduction in the presence of $O_2$ [27]. The inventors have realized that Prussian Blue, which may also be denoted as a ferric hexacyanoferrate, is particularly suited for the electrochemical sensor assay as described herein, as it can be readily applied onto the electrodes and provides for a highly sensitive readout in the differential electrochemical methods.

However, also other analyte-sensitive materials may be provided and/or applied on the electrode for detecting hydrogen peroxide.[28]

In a further preferred embodiment, the sensing electrode comprises a metal hexacyanoferrate preferably selected from the group comprising copper, nickel, cobalt, chromium, vanadium, ruthenium and manganese hexacyanoferrates.

In a further preferred embodiment, the sensing electrode comprises metallophthalocyanines and/or metalloporphyrins such as cobalt phthalocyanine, cobalt tetraruthenated porphyrin, ether-linked cobalt phthalocyanine-cobalt tetraphenylporphyrin or iron phthalocyanine.

In a further preferred embodiment, the sensing electrode comprises a heme protein preferably selected from the group comprising horseradish peroxidase (HRP), catalase (CAT), cytochrome c (Cyt c), hemoglobin (Hb), microperoxidase (MP) and myoglobin (Mb). Heme proteins preferably refer to a category of metalloproteins containing iron centered porphyrin as their prosthetic groups.

In a further preferred embodiment, the sensing electrode comprises CNTs or graphenes. Studies have shown that CNTs can electrocatalyze both the oxidation and reduction of $H_2O_2$. Similarly, the redox capability of graphenes may be exploited to detected hydrogen peroxide.[28]

In a further preferred embodiment, the sensing electrode comprises metal or metal oxides as an analyte-sensitive material, preferably for the detection of hydrogen peroxide.

Metals, in particular transition metals and their compounds have been described as suited catalysts either because of their ability to adopt multiple oxidation states or, in the case of the metals, to adsorb other substances onto their surface and activate them in the process. A wide range of transition metals including platinum (Pt), palladium (Pd), copper (Cu), rhodium (Rh), iridium (Ir), ferrum (Fe) and Gold (Au) have been successfully used for electrocatalyzed $H_2O_2$ determination and may thus constitute suitable analyte-sensitive materials. In preferred embodiments the transition metals may be provided as nanomaterials with different shapes and structures and/or nanoparticles. Such nanomaterial yield particularly confident measurement results due to an increase of the effective surface. In some embodiments they may also be used to modify carbon pastes.

Also, metal oxides, in particular transition metal oxides such as manganese oxide, cobalt oxide, titanium dioxide, copper oxide and iridium oxide show a good electrocatalytic activity to $H_2O_2$, making them a preferred analyte-sensitive material for the detection of hydrogen peroxide.

The electrochemical sensor is characterized in that the salt immobilized in said support is hydrophilic to an extent that the humidity of human exhaled breath is sufficient to form a conductive electrolyte. A hydrophilic salt is attracted to water molecules and tends to be dissolved by water. Typically, the interactions with water and other polar substances are thermodynamically more favorable than their interactions with oil or other hydrophobic solvents. The more hydrophilic the salt is, the better it will dissolve in the presence of exhaled breath of a human and thus provide for a conductive electrolyte usable for the electrochemical detection as described herein.

The electrochemical sensor is further characterized in that the salt immobilized in said support is hygroscopic to an extent sufficient to keep the porous substrate material wet. Hygroscopic salts ensure an efficient absorption of humidity or moisture from breath of a human or animal. Thereby it can be ensured that the support stays wet or moist during multiple exhaling and/or inhaling cycle, which ensures a confident detection. An undesired local drying of the substrate, which may distort the measurement can be efficiently prevented.

In a further preferred embodiment of the invention the electrochemical sensor is characterized in that the salt immobilized in said support is selected from the group consisting of potassium chloride, sodium chloride, sodium acetate, ammonium acetate and monosodium phosphate, most preferably the salt is potassium chloride. The aforementioned types of salts, in particular the most preferred potassium chloride, have proven to be hydrophilic to an extent that the humidity of human exhaled breath is sufficient to form a conductive electrolyte and hygroscopic to an extent sufficient to keep the porous substrate material wet. The preferred salts are thus particularly suited to provide electrolytes for a precise electrochemical detection.

In a further preferred embodiment of the invention the electrochemical sensor is characterized in that the salt is immobilized in the support by applying a solution containing the salt on the porous material and letting said solution dry. The method for immobilizing the salt is simple and surprisingly efficient for a wide range of salts, in particular for the most preferred porous materials such as paper or cellulose. Furthermore, by using a solution as described herein a homogeneous distribution of the salt at a desired concentration can be achieved such that a reproducible measurement results at high precision.

In a further preferred embodiment of the invention the electrochemical sensor is characterized in that a mixture of buffer salts is immobilized in the support by applying a buffer solution containing a respective mixture of salts on the porous material and letting said solution dry. In preferred embodiments, a phosphate buffer solution may be used. The method for immobilizing the salt or mixture of buffer salts is simple and surprisingly efficient for a wide range of salts, in particular for the most preferred porous materials such as paper or cellulose. Furthermore, by using a buffer solution as described herein a homogeneous distribution of the salt at a desired concentration can be achieved such that a reproducible measurement results at high precision.

The electrochemical sensor comprises at least one pair of working electrodes, at least one counter electrode and optionally, one reference electrode.

For the electrochemical sensor , two working electrodes are employed, wherein one sensing electrode will provide an analyte-specific signal, while the blank-electrode may account for non-specific background signals. Hereby, a differential amperometric detection method can increase the accuracy as described herein.

The change in electrical current between the two working electrodes and a counter electrode may be determined for example by applying a voltage of known value. In the presence of an analyte an increase current for the sensing vs. the blank electrode may be detected and allows to determine the presence or concentration of the analyte as described herein. In order to have a more absolute knowledge of the potential of the working electrode the potential of the working electrodes may be set relative to an optional reference electrode, wherein the circuit is completed by the counter electrode. The reference electrode typically does not pass current.

Since (bio)electrochemical reactions are generally detected only in close proximity to the electrode surface, the electrodes themselves have a role in the performance of electrochemical biosensors. Based on the chosen function of a specific electrode, the electrode material, its surface modification or its dimensions may be optimized to enhance the detection ability. The electrodes should be preferably conductive and chemically inert. For instance, platinum, gold, carbon (e.g. graphite) and silicon compounds can be used depending on the analyte.

In a further preferred embodiment of the invention the electrochemical sensor is characterized in that the electrochemical sensor comprises a silver/silver chloride reference electrode and/or a carbon counter electrode.

In a further preferred embodiment of the invention the electrochemical sensor is characterized in that the at least two working electrodes are carbon, platinum, gold or silver electrodes, preferably producible by printing a suitable paste, e.g. a carbon, platinum, gold or silver paste, onto and/or into the support.

The use of conductive paste as a material for the electrodes has proven a particularly effective method for integrating the electrodes into the support. In particular screen printing techniques may be utilized to precisely position the electrodes in respect to the support. Beneficially a functionalization of the working electrodes, i.e. as a sensing and a blank electrode may also be achieved during the screen printing omitting the need of separate manufacturing steps. For instance, to this end the conductive pastes, such as a carbon paste may comprise an analyte-sensitive material such as Prusssian blue.

In a further preferred embodiment of the invention the electrochemical sensor is characterized in that the electrochemical sensor comprises at least two or more analyte-sensitive sensing electrodes directed at the detection of one or more, preferably two or more analytes. In some embodiments it may thus be preferred to use a single analyte-insensitive blank electrode for multiple sensing electrodes for a differential electrochemical detection.

However, in other embodiments it may be preferred to use a separate blank electrode for each additional sensing electrode. In a further preferred embodiment of the invention the electrochemical sensor is thus characterized in that the electrochemical sensor comprises at least two or more pairs of working electrodes directed at the detection of one or more, preferably two or more analytes.

A particular advantage of the electrochemical cell and measurement procedure as described herein lies in its capacity for a scale up. By integrating two or more pairs of working electrodes into the support, e.g. using screen printing techniques as described herein, the analysis of multiple analytes with the breath of a subject is possible and thus allows for a particular comprehensive human breath analysis.

In a further preferred embodiment of the invention the electrochemical sensor is characterized in that a structured pattern of a hydrophobic material, preferably a wax, is applied, preferably printed, onto the porous support material, thereby preferably forming an impermeable barrier inside the porous substrate material after final processing (for example, by baking).

In a further preferred embodiment of the invention the electrochemical sensor is characterized in that the structured pattern comprises an opening forming an electrochemical cell in which the at least one pair of working electrodes and the at least one reference electrode and/or counter electrode are located.

In a further preferred embodiment of the invention the electrochemical sensor is characterized in that the structured pattern may include different compartments inside the electrochemical cell in which the support and/or an electrode are sensitized for the analyte by coating and/or functionalization, wherein preferably an ion flow between the compartments is possible, but which may also act as diffusion barrier.

Using a structured pattern of a hydrophobic material, preferably a wax, allows for a precise shaping of desired compartments of an electrochemical cell on the substrate. In parts that are covered with a hydrophobic material, the liquid portion of the breath is preferably repelled, such that these areas of the substrate are not wetted and do not take part of the electrolytic reactions. Instead, compartments for the electrochemical reactions can be precisely defined and optimized for an analyte detection. In some embodiments a PMMA or other insulating materials with an adhesive foil may be used to further seal the areas outside of the electrochemical cell and/or define distinct compartments.

Such a structuring on top of a porous material is furthermore easily implemented and can be combined in a straightforward manner with screen printing techniques for applying conductive tracks and/or electrodes keeping production costs low.

In a further preferred embodiment of the invention the electrochemical sensor additionally comprises a processing unit configured for the reading of electrical signals, preferably amperometric signals of said electrodes and processing of said signals to monitor the presence of the analyte.

The data processing unit is preferably a unit suitable and configured for receiving, transmitting, storing and/or processing data, preferably of signals from the electrodes or other measurement data. The data processing unit preferably comprises an integrated circuit, a processor, a processor chip, microprocessor and/or microcontroller for processing data, as well as a data memory, for example a hard disk, a random-access memory (RAM), a read-only memory (ROM) or a flash memory for storing the data.

In order to carry out the processing of said signals to monitor the presence of the analyte and optionally determine its concentration, a software, firmware or computer program, respectively, which comprises commands to carry out the calculation steps, may preferably be stored on the data processing device.

For example, the data processing device may be a microprocessor, which is compactly installable on the electrochemical sensor. But also, a system of an electrochemical sensor with an external processing unit such as a personal computer, a laptop, a tablet, a smartphone or the like is conceivable, which in addition to means for receiving, sending, storing and/or processing data also comprised a display of the data as well as an input means, such as a keyboard, a mouse, a touch screen etc. The expert recognizes that preferred (calculation) steps, which are disclosed in connection with the method, can also be performed preferentially by the data processing unit.

In some embodiments the processing unit may be a microprocessor, whereby all components of the processor are arranged on a microchip or integrated circuit (IC). The microprocessor can preferably also be a microcontroller, which integrates further peripheral elements on the microchip in addition to the processor and also has, for example, a data memory, or communication interfaces for receiving and/or transmitting data to a mobile device.

In a preferred embodiment of the invention the electrochemical sensor additionally comprises a communication interface for receiving and/or transmitting data to a mobile device. Examples of suitable communications interfaces include interfaces based upon Bluetooth, Lightning, USB, WLAN or other standard protocols. With these interfaces data can be received and/or transmitted to a suitable mobile device, including but not limited to smartphones, tablets, notebooks etc.

In a further aspect the invention relates to a breath analysis and/or monitoring system comprising a) an electrochemical sensor as described herein b) a filter extension and/or c) a respiratory mask, wherein the electrochemical sensor is compatible with the filter extension and/or respiratory mask. Compatible preferably means that the electrochemical sensor can be readily integrated and/or installed into a filter extension and/or a respiratory mask.

In a preferred embodiment the invention relates a filter extension for a respiratory mask comprising an electrochemical sensor as described herein.

In a further preferred embodiment, the invention relates to a respiratory mask comprising a filter extension in which an electrochemical sensor is incorporated.

A breath analysis of the prior art, e.g. for detecting hydrogen oxide, is typically employed on exhaled breath condensate (EBC). Instead the electrochemical sensor as described herein allows for a direct analysis of the human breath. When implemented into a breath analysis and/or monitoring system comprising a filter extension and/or a respiratory mask the electrochemical sensor can be readily positioned into the breathing path of the subject. Wearing a respiratory mask equipped with an electrochemical sensor as described herein allows for a continuous monitoring of analytes directly from the exhaled breath.

The term respiratory mask has the typical meaning in the relevant field, and preferably refers to a mask with attachment means for positioning the mask into the respiratory flow of a subject, in particular in front of the nose and/or mouth. Typically, respiratory mask may comprise a filter for filtering particles or substances from the air. Respiratory mask is typically used for either protecting the subject from the environment, e.g. from dusts and airborne microorganisms, as well as hazardous fumes, vapors and gases or vice versa protecting the environment or third person from the subject, as for instance in case of a surgeon masks.

Common respiratory masks include filter extension, which refer to a component for holding and/or positioning a filter. Thereby for instance, a filter can be exchanged, while the main components of a respiratory mask may be reused.

Since the electrochemical sensor as described herein may be flexibly designed and dimensioned, the sensor can in preferred embodiments be shaped in order to fit into a filter extension or other suitable component of a respiratory common mask or be directly applied onto a common mask.

The embodiment is particular preferred for point-of-care applications, wherein a low-cost, wearable electrochemical sensor readily positioned for a breath analysis can be provided and used a continuous monitoring of desired analytes directly from the exhaled breath.

In a further aspect the invention relates to a method for an on-site or clinical monitoring of the presence of an analyte in the breath of a subject comprising

    a. Providing an electrochemical sensor as described herein.

    b. Positioning said electrochemical sensor in the respiratory flow of said subject.

    c. Employing a differential measurement by detecting the differential electrochemical signal at the at least one pair of working electrodes in order to monitor the presence of the analyte.

Technical features that have been disclosed for the electrochemical sensor as described herein also apply for the breath analysis and/or monitoring system or method for monitoring the presence of an analyte in the breath of a subject. A person skilled in the art recognizes that preferred features of the electrochemical sensor as described herein can be advantageously employed in the context of the breath analysis and/or monitoring system and/or method for monitoring the presence of an analyte in the breath of a subject and convey the same beneficial effects.

In a preferred embodiment of the invention the method is characterized in that the presence of the analyte is monitored continuously during a single or multiple exhaling and inhaling cycles.

In a further preferred embodiment of the invention the method is characterized in that different segments of the monitored signal can be used in order to quantify the presence of the analyte in different regions of the lung and/or airways.

In a further preferred embodiment of the invention the method is characterized in that the signal detected at the analyte-insensitive blank electrode is used for a background correction of non-specific interferences of the signal detected at said analyte-sensitive sensing electrode.

Herein it is particularly, preferred that the background correction method is able to compensate current variations caused by the respiratory movement (due to variation of air flow, humidity, temperature, etc.) and environmental conditions.

By using the electrochemical sensor with the beneficial differential detection method as described herein the method allows for a precise monitoring of the presence and/or concentration of an analyte. An implementation of the electrodes into a porous substrate, which as described herein can be designed hygroscopic and air-permeable further ensures a comfortable breathing during the monitoring which makes the method particularly suited also for long-term observations.

## DETAILED DESCRIPTION OF THE INVENTION

The invention relates to an electrochemical sensor comprising a porous support as well as at least one pair of working electrodes, a sensing and a blank electrode, which are applied onto and/or integrated into said porous supporting material, wherein a salt is immobilized in said support, such that upon exhaling onto the sensor a differential electrochemical measurement at said pair of working electrodes allows for monitoring the presence of the analyte in the breath of said subject. The invention further relates to a breath analysis or monitoring system comprising said electrochemical sensor well as methods for using the same.

Sensor devices, based on the differential measurement approach described herein, on porous substrates (such as a paper based systems) allow for low-cost, direct and long-term breath testing of different analytes. They can be fabricated to be lightweight for the user's comfort and contain nontoxic electrolytes and electrode material for the user's safety. The implemented breath sensing system can also be extended with a compact and low-power wearable signal readout unit along with a mobile app to enable on-site monitoring. Hence, the subjects, for example, with chronic respiratory diseases, do not have to visit the doctor's offices for the required frequent check-ups to monitor the course of the disease.

The term "analyte" refers to a substance to be detected, quantified or otherwise assayed by the method of the present invention. Typical analytes may include, but are not limited to hydrogen peroxide, glucose, pathogens, proteins, peptides, nucleic acid segments, carbohydrates, lipids, antibodies (monoclonal or polyclonal), antigens, oligonucleotides, specific receptor proteins, ligands, molecules, cells, microorganisms and fragments and products thereof, genetic materials, hormones, hydrocarbons, aldehydes, sulfides, ammonia, ethanol, acetone, isoprene, ethane, carbonyl sulfides, carbon dioxides, carbon monoxide, nitrogen monoxide and volatile organic compounds (VOCs) or any substance for which an analyte sensitive-material for the electrode or the support can be developed or provided to act as a catalyst or mediator for an electrochemical reaction.

The term "analyzing" or "monitoring the presence of an analyte" as used herein preferably refers to detecting the presence, qualitatively identifying, or quantitatively measuring the amount, concentration, or changes in levels of analytes in the breath of a subject.

The term "subject" includes both human and veterinary subjects.

As used herein "sample" relates to the "breath" of a subject, which refers to the expiratory or respiratory volume exhaled by a subject typically composed of a gas fraction and a liquid fraction.

The term "sensing electrode" or "analyte-sensitive sensing electrode" preferably refer to a working electrode that comprises an analyte-sensitive material which may act as a mediator catalyst for the reaction of the analyte. In some embodiments either the sensing electrode itself or the section of porous substrate in proximity of the sensing electrode contains analyte-sensitive material. For instance, in some embodiments the analyte-sensitive material may be applied onto the electrode. In some embodiments it may be preferred that the sensing electrode is situated in a compartment of the porous substrate, in which analyte-sensitive material has been immobilized.

The immobilization is preferably to be understood in the broadest sense and shall refer to any kind of interaction or binding, which restricts or associates the analyte-sensitive material to the compartment of the porous substrate. The immobilization is preferably chosen depending on the type of analyte-sensitive material. Examples may include allowing for an adsorption of the analyte-sensitive material (e.g. being diluted in a buffer solution) into the porous substrate, a covalent binding, encapsulation or entrapment in a gel (such as a hydrogel) into the porous substrate In some embodiments the expression immobilizing may refer to a binding such as a covalent, a non-covalent, an ionic or electrostatic binding such as a hydrogen bonding, metal ion-binding, ionic interactions among charged groups, van der Waals interactions, or hydrophobic interactions among non-polar groups.

The term "blank electrode" or "analyte-insensitive sensing electrode" preferably refers to a working electrode that does not comprises an analyte-sensitive material, which may act as a mediator or catalyst for the reaction of the analyte. The phrase not comprising an analyte-sensitive material preferably means that the blank electrode neither contains analyte-sensitive material, nor is situated in a compartment of the porous substrate, in which an analyte-sensitive material has been immobilized. The blank electrode may thus account for an analyte-independent, non-specific background signals, which can be used to increase measurement accuracy by employing a differential electrochemical detection method as described herein.

As used herein "analyte-sensitive material" may refer to any material that can act as a mediator or catalyst for an electrochemical reaction.

The term "catalyst" or "mediator" as used herein describes a molecule which increases the rate of an electrochemical reaction, but which is not necessarily consumed by the reaction.

Electrochemical reaction may relate to any reaction either caused or accompanied by the passage of an electric current and involving in most cases the transfer of electrons.

Electrochemical sensors are well known in the art for use in the detection of components. The sensors generally comprise at least two electrodes, a working electrode and a counter electrode. The change in electrical current signal between the electrodes is determined, for example by applying a voltage of known value and form across the electrodes, as a result of the sensor being brought into contact with a medium that may comprise analytes. Likewise, other electrically measurable signal changes (such as impedance or conductance may be used as known in the art. In many cases, the electrodes are coated with an electrolytic or semi-conductor material that bridges the electrodes, the conductivity of which changes as a result of contact with the analyte.

Electrochemical sensors may also be referred to as amperometric systems. By applying the voltage between two electrodes (a working and a counter electrode) they can oxidize (or reduce) an analyte of interest and use the resulting current to estimate its concentration.

For instance, for detecting hydrogen peroxide at a sensing electrode hydrogen peroxide can be electrochemically converted resulting in a concentration dependent current signal. Hydrogen peroxide can be both oxidized and reduced at the electrode surface. Hydrogen peroxide can then be detected by direct electrochemical conversion at this electrode, which may comprise a platinum electrode acting as an analyte-sensitive material. Preferably the sensing electrode comprises however Prussian Blue, Cobalt phthalocyanine or other molecules for enhancement of selectivity/catalysis, possibly with different electrode material, and the use of nano-/microparticles for an increase of efficiency.

The term "Prussian blue", as used herein, relates to various iron cyanide blue pigments but more specifically to the ferric ferrocyanide. Because of its wide use, Prussian blue may be referred to below as the iron blue or the blue pigment. Typically Prussian blue is a dark blue pigment produced by oxidation of ferrous ferrocyanide salts, which preferably has the chemical formula $Fe_4[Fe(CN)_6]_3$.

The analyte-sensitive material will be chosen according to the detection of the desired analyte. For instance, electrochemical glucose sensors are known that use an enzyme to catalyze the glucose into hydrogen peroxide. The hydrogen peroxide is then re-oxidized by a catalyst or mediator, which is the oxidized species of a redox couple. A common example of a catalyst is ferricyanide. In oxidizing the hydrogen peroxide the e.g. ferricyanide is reduced to ferrocyanide. Sufficient ferricyanide is present that it is always in excess to the amount of ferrocyanide produced. The ferrocyanide may now be oxidized at the working electrode (WE), which has at a positive voltage with respect to the other electrode, generating a current. Another electrode, the counter electrode (CE) may complete the circuit, typically by converting ferricyanide to ferrocyanide. This is called mediated electron transfer. However, the analyte of interest can sometimes be oxidized or reduced electrochemically directly, in which case it can be measured directly by direct electron transfer.

Electrochemical sensing may employ a reference electrode, a counter or auxiliary electrode and a working electrode, also known as the sensing or redox electrode. The reference electrode, for instance a Ag/AgCl electrode, may be kept at a distance from the reaction site in order to maintain a known and stable potential. The working electrode can serve as the transduction element in the (bio)chemical reaction, while the counter electrode establishes a connection to the electrolytic solution so that a current can be applied to the working electrode. These electrodes should be both preferably conductive and chemically inert. For instance, platinum, gold, carbon (e.g. graphite) and silicon compounds can be used depending on the analyte.

For extracting information from biological systems such as a human breath a bio-electrochemical component may serve as a transduction element. As recognition elements for electrochemical detection techniques enzymes may be preferred. This is mostly due to their specific binding capabilities and biocatalytic activity. Other biorecognition elements such as e.g. antibodies, aptamers, nucleic acids, cells and micro-organisms as described herein may also be applied. An immunosensor may use antibodies, antibody fragments or antigens to monitor binding events in bioelectrochemical reactions. Typically, in (bio-)electrochemistry, the reaction under investigation would either generate a measurable current (*amperometric*), a measurable potential or charge accumulation (*potentiometric*) or measurably alter the conductive properties of a medium (*conductometric*) between electrodes or the electrodes itself.

An electrode chemical sensor that uses a working electrode (WE) and a counter electrode (CE) is called a two-electrode system. Sometimes it is desirable to have a more absolute knowledge of the potential of the WE in which case a three-electrode system is used. In this case, the potential of the WE is set relative to a reference electrode (RE) and the circuit is completed via a CE. The RE does not pass current.

For the electrochemical sensor as described herein, two working electrodes are employed, wherein one sensing electrode will provide an analyte-specific signal, while the blank-electrode may account for non-specific background signals. Hereby, a differential electrochemical detection method can increase the accuracy as described herein.

The electrochemical sensor may also relate to an electrochemical detection of analytes using metal, metal oxide-, semiconducting micro- and nanoparticles or redox active substances. The methods employ metal particles (e.g., metal nanoparticles) conjugated to biorecognition elements (such as aptamers). The biorecognition element can be conjugated to the metal particle by any suitable covalent or non-covalent means. After binding to its target, aptamers can change their structure in such way that the conjugated particles are touching the electrode surface and thus, can serve as an electrochemical label for the analyte. The intensity of the resulting current peak reflects the amount of metal oxidized at the working electrode, and therefore the amount of metal particles (and thus, analyte) present.

The particle can be, for example, a metal nanoparticle. A metal particle comprises any suitable metal. Such as gold, silver, copper, platinum, rhodium, palladium, iridium, nickel, iron, bismuth, cadmium, cobalt, or combinations thereof. The metal particle can also comprise a suitable metal compound, such as, for example, a metal oxide, halide, and/or chalcogenide, Such as AgO, Agl, BiOs, CuO, CdP, CdS, CdSe, CdTe. Co-O, CrO, CuS, Hgl, MnO, PbS, PbO, SnO, TiO, RuO, ZnO, ZnS or ZnO. Suitable metal particles can be selected in view of a number of factors, including the nature of the oxidation process employed, the nature of the electrochemical techniques employed, the desired stability of the metal particle towards environmental conditions (e.g., stability in air), and combinations thereof.

Recognition elements for particular analytes are known in the art. An appropriate recognition element for the formation of an analyte conjugate can be selected in view of a number of considerations including analyte identity or analyte concentration,

Suitable recognition elements include antibodies, antibody fragments, antibody mimetics (e.g. engineered affinity ligands), peptides (natural or modified peptides), proteins, (e.g., recombinant proteins, host proteins), polynucleotides (e.g., DNA or RNA, oligonucleotides, aptamers), receptors, ligands, antigens, organic small molecules (e.g., antigen or enzymatic co-factors), and combinations thereof.

In some embodiments, the recognition element selectively associates with the analyte. The term "selectively associates", as used herein when referring to a recognition element, refers to a binding reaction which is determinative for the analyte in a heterogeneous population of other similar compounds. Generally, the interaction is dependent upon the presence of a particular structure (e.g., an antigenic determinant or epitope) on the binding partner. By way of example, an antibody or antibody fragment selectively associates to its particular target (e.g., an antibody specifically binds to an antigen) but it does not bind in a significant amount to other proteins present in the sample or to other proteins to which the antibody may come in contact during a breath analysis.

By "protein", a sequence of amino acids is meant for which the chain length is sufficient to produce the higher levels of tertiary and/or quaternary structure. "Peptides" preferably refer to smaller molecular weight proteins.

The term "antibody" as used herein covers monoclonal antibodies (including full-length monoclonal antibodies), polyclonal antibodies, multi-specific antibodies (e.g., bispecific antibodies), and antibody fragments so long as they exhibit the desired biological activity.

"Antibody fragments" comprise a portion of a full-length antibody, generally the antigen binding or variable region thereof. Examples of antibody fragments include Fab, Fab', F(ab')$_2$, and Fv fragments; diabodies; linear antibodies; single-chain antibody molecules; and multi-specific antibodies formed from antibody fragments.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see, e.g., U.S. Pat. No. 4,816,567). The "monoclonal antibodies" may also be isolated from phage antibody libraries using the techniques described in Clackson et al, Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. NatL. Acad Sci. USA 81:6851-6855 (1984)).

The analyte-sensitive material may thus also relate to analyte-sensitive receptors, such as antibodies, enzymes, binding proteins, molecularly imprinted polymers, nucleic acids, aptamers, which cause signal change in dependence of the analyte concentration.

The expression "support" as used herein refers to the portion of the electrochemical sensor which serves as the "substrate" or "support material" onto which the electrodes may be applied or integrated into.

A suitable support according to the present invention should be mechanically stable, flexible, hygroscopic and air-permeable, chemically and mechanically stable over a relevant pH range and temperature range.

Typically, the support may be provided as a flat sheet of the porous substrate material, i.e. in a form with a substantially reduced thickness, compared to its width or length. Preferred dimensions of a support may include a thickness of less than 1 cm, preferably less than 1 mm, a width of in between 2 mm and 100 mm, preferably 5 to 20 mm and a length of 2 mm to 100 mm, preferably 10 mm to 50 mm.

The support is made of a porous substrate material. Porosity is preferably a measure of the void (i.e. "empty") spaces in a material and can be for instance expressed as a fraction of the volume of voids over the total volume.

As used herein, the term "porous material" preferably refers to a material which is permeable such that fluids or gases such as air are movable there through by way of pores or other passages. An example of a porous material is a cellulosic material. Other examples of porous materials include hydrogel, hydrophilic polymers, ceramics, stone, concrete, and derivatives thereof. As used herein, the term "cellulosic material" refers to a material that includes cellulose as a structural component. Examples of cellulosic materials include wood, paper, textiles, rope, particleboard and other biologic and synthetic materials. As used herein, wood includes solid wood and all wood composite materials (e.g., chipboard, engineered wood products, etc.). Cellulosic materials generally have a porous structure that defines a plurality of pores.

Preferred porous materials are cellulose based materials (like paper), ceramics, a hydrogel and/or a hydrophilic polymer.

As used herein, the term "polymer" refers to a molecule composed of repeating structural units typically connected by covalent chemical bonds. The term "polymer" is also meant to include the terms copolymer and oligomers.

The expression "integrated into the support" preferably relates to an at least partial immersion of the (working) electrodes in the support made of a porous material. Preferably, to this end part of material of the electrodes are brought into the porous material, e.g. filling the gaps and/or voids of the porous material. To this end, an electrode may for instance be applied onto the substrate as conductive paste, e.g. carbon paste, which may submerge into the substrate.

To allow for a detection of a current between the electrodes that reflects an analyte-dependent electrochemical reaction furthermore a salt is immobilized within the support.

In chemistry, a salt is a chemical compound consisting of an assembly of cations and anions. Salts are composed of related numbers of cations (positively charged ions) and anions (negative ions) so that the product is electrically neutral (without a net charge). These component ions can be inorganic, such as chloride (Cl-), or organic, such as acetate ($CH_3CO_2^-$); and can be monatomic, such as fluoride (F-), or polyatomic, such as sulfate ($SO_4^{2-}$).

In dry form salts are typically insulators, when brought into contact with a liquid salts dissolve and the solutions containing dissolved salts (e.g., sodium chloride in water) serve as electrolytes.

The dissolved electrolyte separates into cations and anions, which disperse uniformly through the solvent. Electrically, such a solution is neutral. If an electrical potential is applied to such a solution, the cations of the solution are drawn to the electrode that has an abundance of electrons, while the anions are drawn to the electrode that has a deficit of electrons. The movement of anions and cations in opposite directions within the solution amounts to a current.

In some embodiments a salt or a salt mixture, for example a buffer mixture, is immobilized in said support by applying a solution comprising a salt or salt mixture onto the porous material and allow the solution to dry. For instance, a solution comprising 1 mM to 100 M, preferably 0.1 M to 10 M of a salt or salt mixture may be provided and applied onto the porous substrate. By immersion into the pores of the substrate, the salts may be immobilized.

Preferably examples of a salt include but are not limited to potassium chloride, sodium chloride, sodium acetate, ammonium acetate, monosodium phosphate and buffer salt mixtures, for example, phosphate buffer, most preferably the salt is potassium chloride. Beneficially for these salts the humidity of a human breath is sufficient to form electrolytic solutions within the support such that the electrochemical reactions can take place as described herein.

In some embodiments it may also be preferred to apply ionic liquids to the substrate in order to immobilize a salt therein. An "ionic liquid" or "IL" is preferably a salt, which at room temperature (25°) is in the liquid state. Such ionic liquids may also be referred to as RTIL (room temperature ionic liquids) In some embodiments the term may refer to salts whose melting point is below 100°C. While ordinary liquids such as water and gasoline are predominantly made of electrically neutral molecules, ionic liquids are largely made of ions and short-lived ion pairs. In the literature ionic liquid may also be called liquid electrolytes, ionic melts, ionic fluids, fused salts, liquid salts, or ionic glasses. Upon applying an ionic liquid to the porous substrate, the ionic liquid may submerge with the substrate such that a salt (in this case in a liquid state) is immobilized within the substrate.

As used herein the term "immobilized" when referring to the immobilization of a salt to a porous substrate is therefore to be understood in the broadest sense and preferably refers to the salts being by any kind of interaction or binding restricted or associated with the porous substrate. Preferably, the salt may be immobilized by simply adding a solution comprising the salt to the porous substrate or by applying an ionic liquid to the substrate.

As used herein the immobilization of the salt shall encompass any kind of association or binding that may occur by such an application of a solution comprising a salt or ionic liquid and is not to be understood as limited to specific types of binding. However, in some embodiments the expression immobilizing may refer to a binding such as a covalent, a non-covalent, an ionic or electrostatic binding such as a hydrogen bonding, metal ion-binding, ionic interactions among charged groups, van der Waals interactions, or hydrophobic interactions among non-polar groups.

In some embodiments a spatial confinement of electrolytic active compartments can be obtained using a structured pattern of hydrophobic surfaces, which preferably reduce the wetting behaviour.

As used herein the term "hydrophobic material" preferably characterize a material that after being applied onto the substrate exhibits a contact angle for water of greater than 90°, which means that the water droplet does not wet the surface.

As is well known in the art, the contact angle can be used as a measure of the wetting behavior of a surface. If a liquid spreads completely on the surface and forms a film, the contact angle is zero degrees (0°). As the contact angle increases, the wetting resistance increases, up to a theoretical maximum of 180°, where the liquid forms spherical drops on the surface. The term hydrophobic may be used as term used to describe a wetting resistant surface where the reference liquid is water.

Different hydrophobic materials can be used, including but not limited to fluorinated polymers, in particular polytetrafluoroethylene, natural and synthetic waxes, for example carnauba wax, paraffin wax, beeswax, polyethylene waxes, polypropylene waxes, Fischer-Tropsch waxes, as well as polymers and copolymers of a-olefins or of cycloolefins (including in particular COC) or heavy silicone oils, for example polymers of polydimethylsiloxane. Waxes are particularly preferred as they can be easily applied in a structured pattern on the substrate.

The electrochemical sensor, the method, system may in some embodiments comprise and/or employ one or more processing units and/or conventional computing devices having a processor, an input device such as a keyboard or mouse, memory such as a hard drive and volatile or nonvolatile memory, and computer code (software).

The components of the computing devices may be conventional, although the device may be custom-configured for each particular implementation. The computer code to perform steps of the method described herein may be written in any programming language or model-based development environment, such as but not limited to C/C++, C#, Objective-C, Java, Basic/VisualBasic, MATLAB, Simulink, StateFlow, Lab View, or assembler.

The information processed and/or produced by the method, i.e. as digital representations of signals stemming from the electrodes, can employ any kind of file format which is used in the industry. Any suitable computer readable medium may be utilized. The computer-usable or computer-readable medium may be, for example but not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a transmission media such as those supporting the Internet or an intranet, cloud storage or a magnetic storage device.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of" and "consisting essentially of". Thus, the terms "comprising"/"including"/"having" mean that any further component (or likewise features, integers, steps and the like) can/may be present. The term "consisting of" means that no further component (or likewise features, integers, steps and the like) is present.

## FIGURES

The present invention is further described by reference to the following figures. The figures exemplify non-limiting and potentially preferred embodiments, presented for further illustration of the invention.

_Description of the figures:_

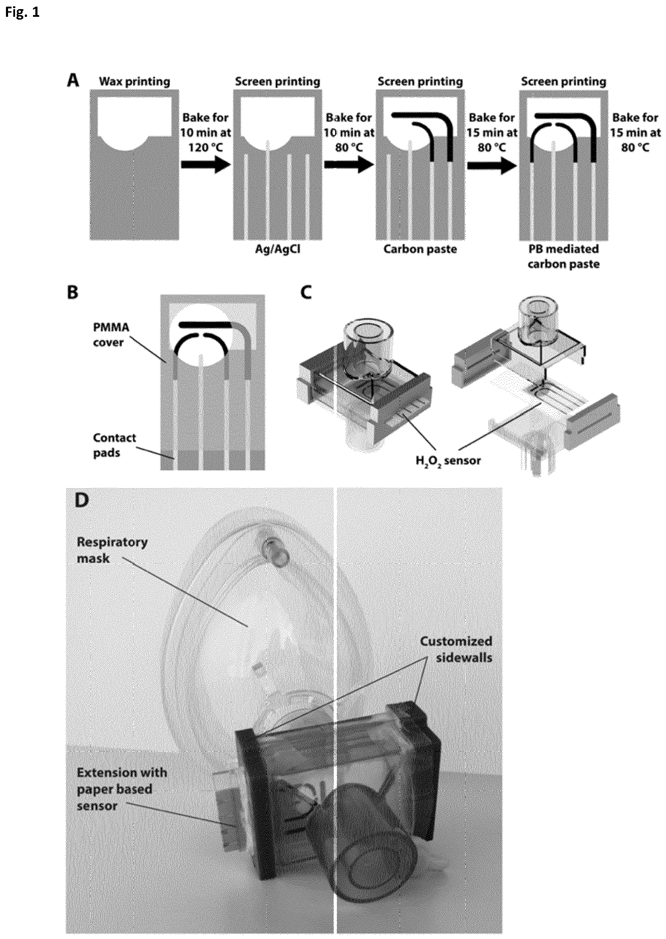

**Figure 1 (A)** Schematics of chip fabrication steps including the wax isolation and the screen printing of the Ag/AgCl, the carbon and the PB-mediated electrodes, **(B)** CAD drawing of the electrochemical sensor with PMMA carrier, **(C)** SolidWorks™ model of a filter extension for respiratory mask, including the paper based hydrogen peroxide sensor and **(D)** image of respiratory mask with the commercial filter extension with customized sidewalls, containing the sensor chip.

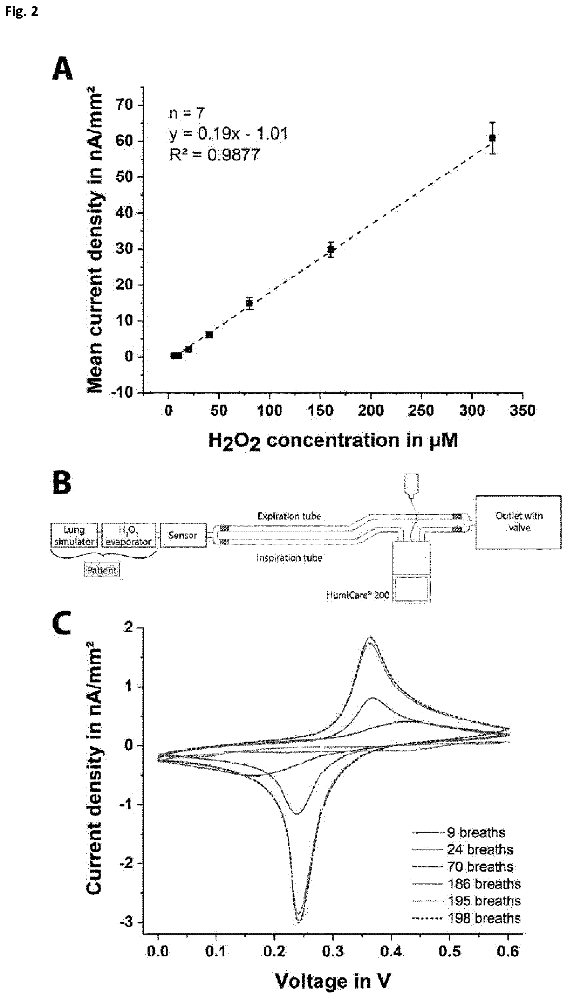

**Figure 2. (A)** Calibration curve of the paper based $H_2O_2$ sensors with different hydrogen peroxide concentrations: 5 to 320 $\mu$M $H_2O_2$ in 1 M KCl solution. Herein, the frontside of the chip was insulated with an adhesive tape since the sensor is placed into the filter with the backside towards the patient and thus, the frontside of the electrodes has no direct contact with the exhaled breath. Error bars represent $\pm$ standard deviation (SD) of n = 7 replicates. **(B)** Scheme of measurement setup for simulation of respiration, including lung simulator, humidifier, $H_2O_2$ evaporator and filter housing with integrated $H_2O_2$ sensor. **(C)** Cyclic voltammograms of a dry chip with a PB coated working electrode, pre-treated with 1 M KCl, in vapor after 9 (grey), 24 (red), 70 (blue), 185 (green), 195 (orange) and 198 (black, dashed) breaths at a scan rate of 100 mV s$^{-1}$.

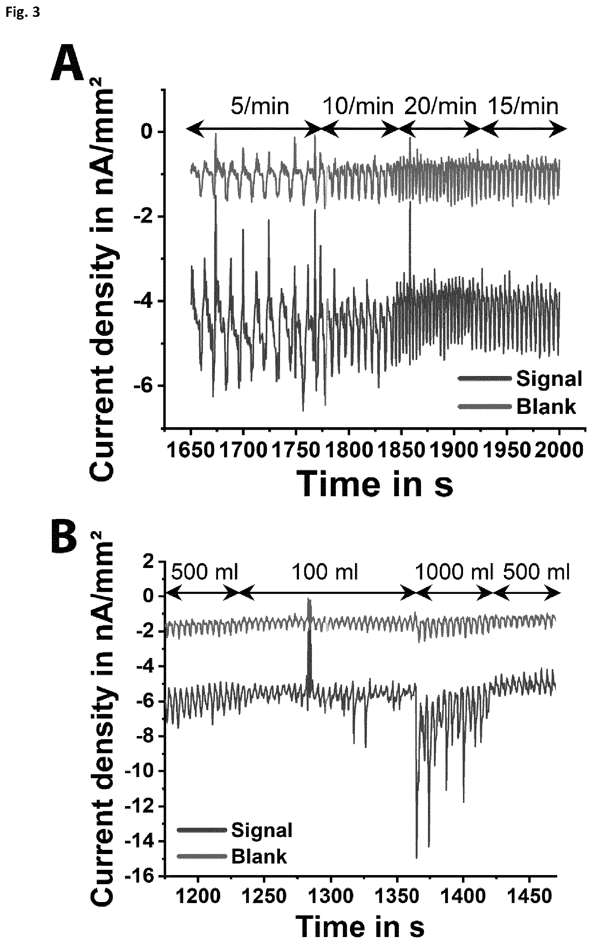

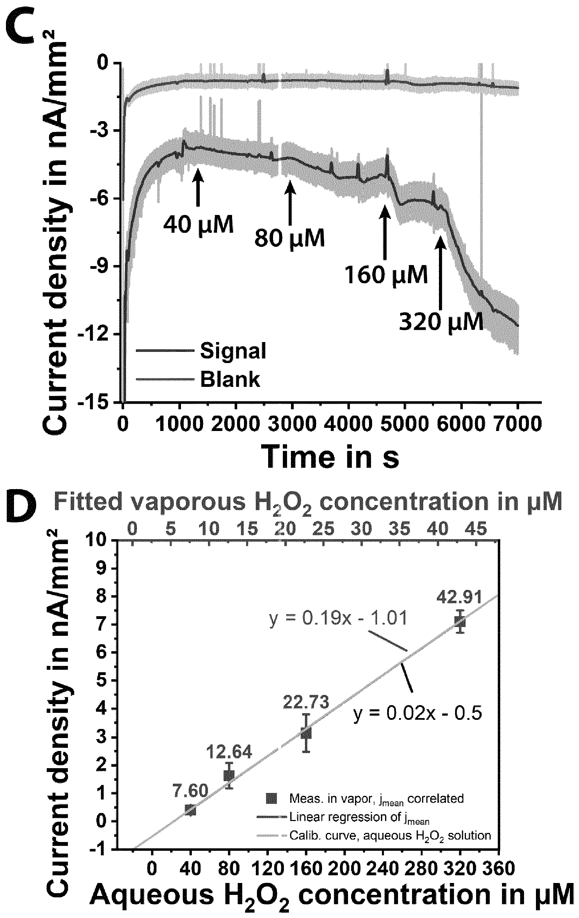

**Figure 3.** Signals of sensing (black) and blank (red) electrodes of an amperometric measurement at different respiration **(A)** frequencies and **(B)** volumes, **(C)** Current density of a calibration measurement with 5 to 320 $\mu$M $H_2O_2$ in vapor, **(D)** calibration curve of the aqueous and vaporous hydrogen peroxide in artificial breath. Error bars represent $\pm$ SD of n = 3 replicates.

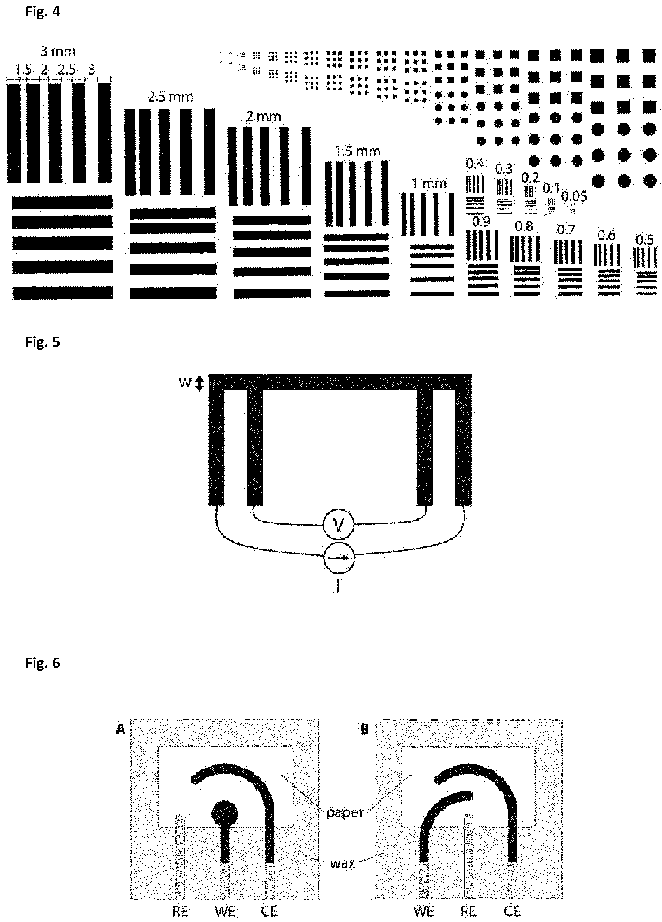

**Figure 4.** CAD drawing of structures used for resolution testing of screen-printing process, containing lines with different widths and distances (0.05 to 3 mm / 0.05 to 1.5 mm) and arrays of circles and squares with different diameters and edge lengths (0.05 to 3 mm).

**Figure 5.** Scheme of structure and configuration for the resistance measurement with 4-point probes method to find the minimum width possible for the conducting paths. The current was applied to the outer legs and the voltage was measured at the inner legs of the structure.

**Figure 6.** CAD drawings of two tested electrode designs with the same 2D area. Electrode design 1 (A) with a smaller edge area, compared to design 2 **(B).** The paper window constitutes the electrochemical cell, where the electrolyte droplet is placed for the measurements. The wax isolation prevents the electrolyte to spread all over the sensor.

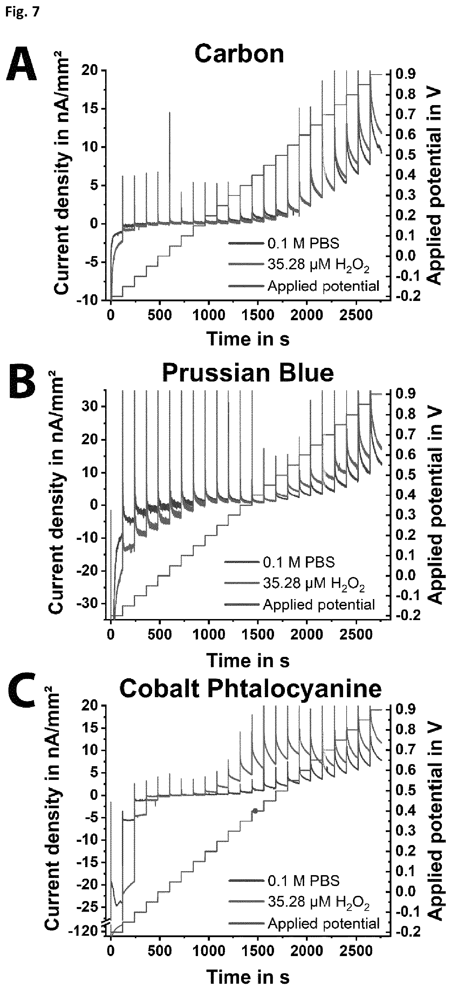

**Figure 7.** Results of multi-step amperometry for **(A)** carbon, **(B)** PB and **(C)** CP mediated carbon electrodes in 0.1 M PBS and 35 $\mu$M $H_2O_2$ at voltages in the range from -0.2 to 0.45 V vs. Ag/AgCl in 0.05 V steps. For PB and CP mediated electrodes, the highest signal difference between PBS and measured $H_2O_2$ concentration was observed at a voltage of 0.0 and 0.4 V, respectively. In the case of the carbon electrode, there was no significant signal change for $H_2O_2$ at the voltages of interest.

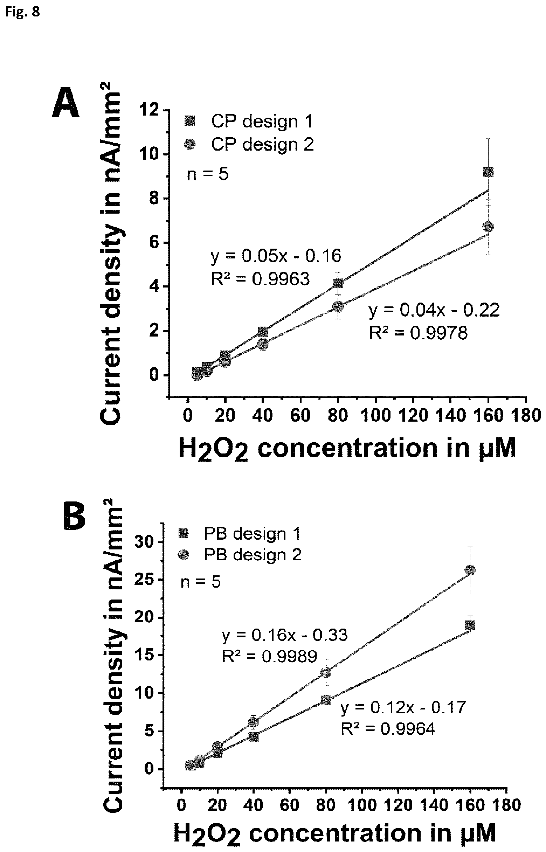

**Figure 8.** Calibration curves of different electrode designs with **(A)** CP and **(B)** PB mediated paste for different $H_2O_2$ concentrations. For the CP mediated paste, design 1 had the higher sensitivity, compared to design 2. Overall, the best results were achieved with PB, where design 2 had the highest sensitivity. Error bars represent $\pm$ SD of n = 5 replicates.

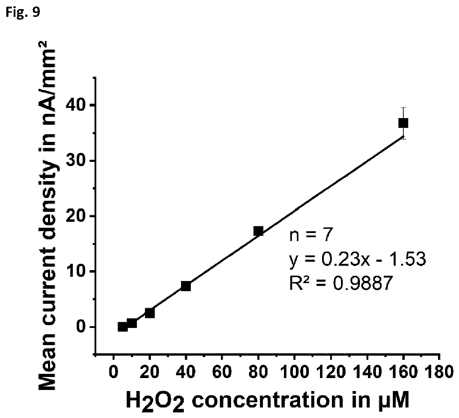

**Figure 9.** Calibration curve of differential electrode design for $H_2O_2$ concentrations between 5 and 160 $\mu$M in 1 M KCl solution applied to the front of the sensor chip. For this calibration, the whole 3D area of the working electrode is in contact with the sample solution. Error bars represent $\pm$ SD of n = 7 replicates.

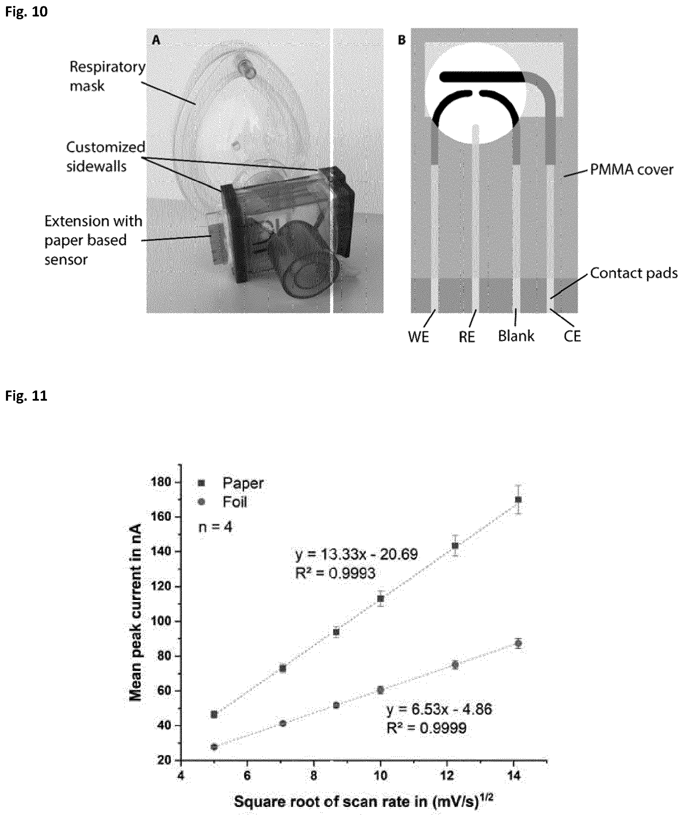

**Figure 10. (A)** Image of respiratory mask with extension with customized 3D printed sidewalls, containing the paper based $H_2O_2$ sensor and **(B)** CAD drawing of differential electrode design with a hydrogen peroxide sensing working electrode (WE), consisting of PB-mediated carbon, a carbon blank electrode (Blank) to subtract background, a silver / silver chloride reference electrode (RE), a carbon counter electrode (CE) and a PMMA cover for stabilization and isolation of conducting tracks from humidity.

**Figure 11.** Plot of mean peak current over square root of scan rate for the screen-printed electrodes on paper and foil in order to determine the electrochemically active electrode area. Error bars represent $\pm$ SD of n = 4 replicates.

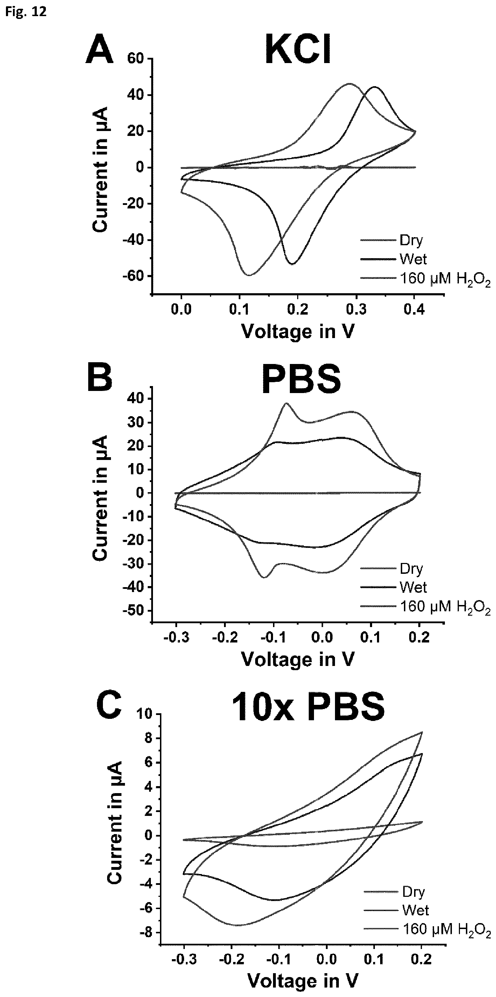

**Figure 12.** Cyclic voltammograms performed with chips under dry and wet condition and with 160 $\mu$M $H_2O_2$ at a scan rate of 100 mV s$^{-1}$ in **(A)** 1 M KCl, **(B)** 0.1 M PBS and **(C)** 10$\times$ PBS. Please note that the results are given in current values, instead of current density, since the electrochemically active surface area of the dry sensor is undefined.

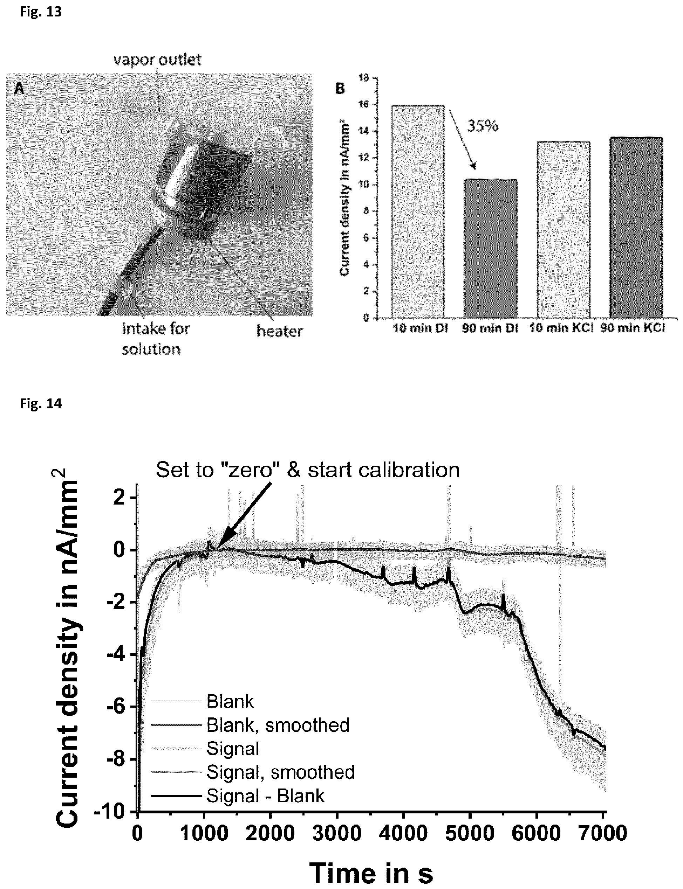

**Figure 13. (A)** Image of humidifier, used for hydrogen peroxide evaporation, with heater element, intake tube and vapor outlet and **(B)** resulting current densities of the stability tests with 80 $\mu$M hydrogen peroxide diluted in DI water and 1 M KCl after 10 and 90 min at 0.0 V *versus* Ag/AgCl, where for DI water a significantly higher decrease can be observed, than for potassium chloride.

**Figure 14.** Offset corrected current density of a calibration measurement using 5 to 320 $\mu$M hydrogen peroxide in vapor.

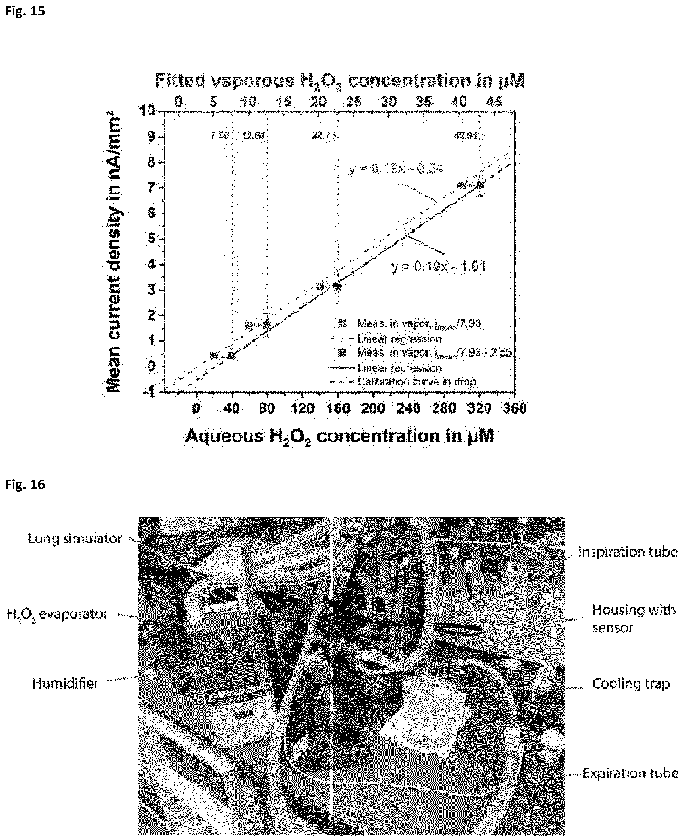

**Figure 15.** The detailed plot showing the correlation of the calibration curves of the aqueous and vaporous hydrogen peroxide measurement in artificial breath. Error bars represent $\pm$ SD of n = 3 replicates.

**Figure 16.** Image of measurement setup employed for exhaled breath analysis, including the lung simulator, the $H_2O_2$ evaporator, humidifier, heated inspiration and expiration tubes, the housing with the sensor and a cooling trap.

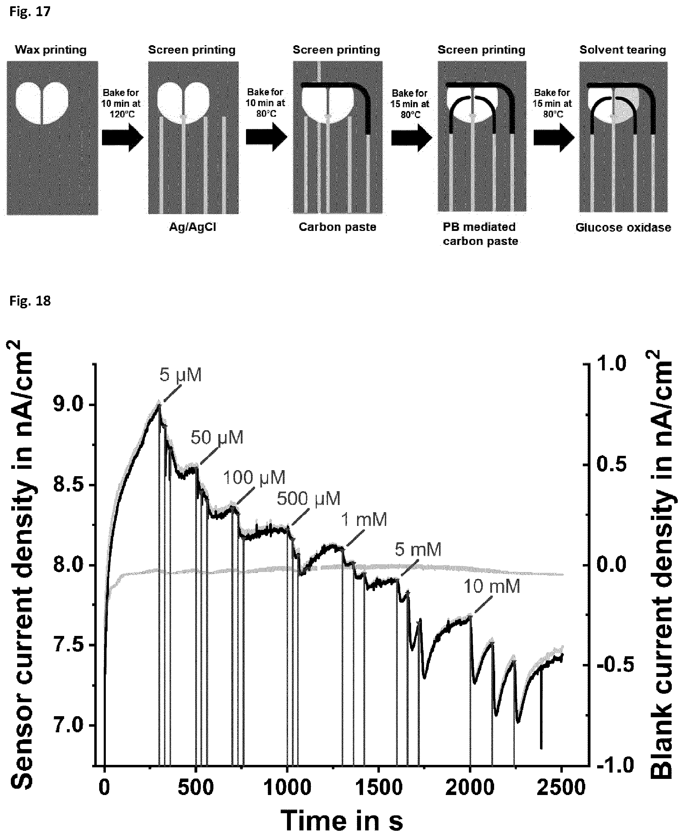

**Figure 17:** Chip design and fabrication showing the main elements of a preferred paper-based glucose sensor: including a wax isolation (lime green), electrodes from left to right: sensing, reference, blank and counter electrodes.

**Figure 18:** Proof-of-principle study of non-invasive glucose monitoring using a paper-based electrochemical sensor. Sensor (black), blank (grey) and differential (green) current density signals (at -0.2 V vs. 1 M Ag/AgCl) during a series of applications of glucose containing aerosol puffs. For each given concentration, three consecutive puffs were applied (red triangles and dotted lines).

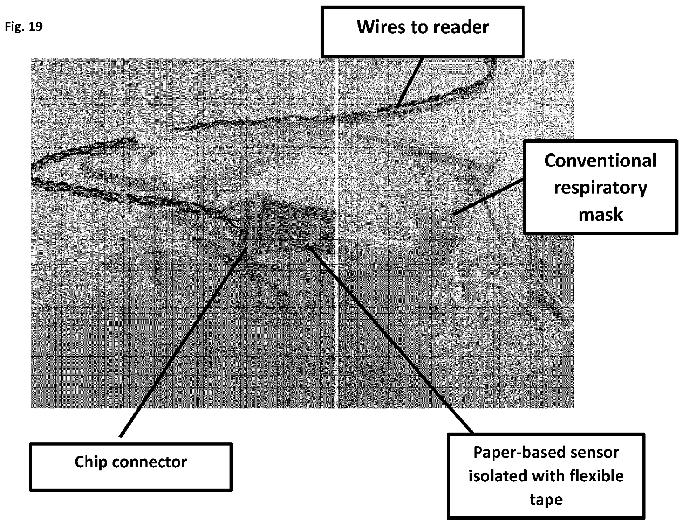

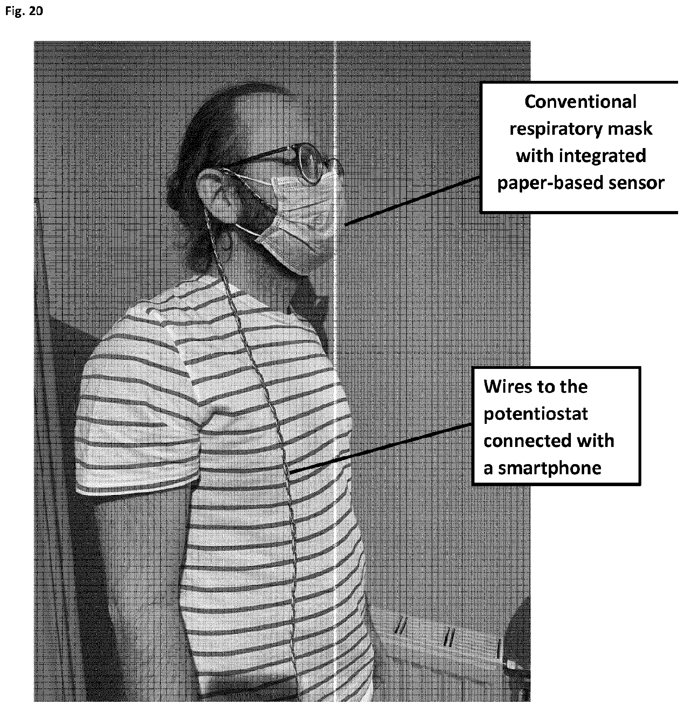

**Figure 19** and **20:** Images illustrating the integration of a preferred paper-based sensor into a conventional respiratory mask.

## EXAMPLES

The invention is further described by the following examples. These are not intended to limit the scope of the invention, but represent preferred embodiments of aspects of the invention provided for greater illustration of the invention described herein.

The following examples report a low-cost approach for the continuous, real-time and on-site surveillance of the concentration of $H_2O_2$ in exhaled breath. The wearable system developed employs a paper based electrochemical sensor (abbreviated in the following as paper sensor) comprising a differential electrode design with a Prussian Blue (PB)-mediated carbon electrode for $H_2O_2$ detection and carbon blank electrode for subtracting the background signals. A silver/silver chloride (Ag/AgCl) reference and carbon counter electrode are used to complete the electrolytic cell. The signal detection is achieved as $H_2O_2$ oxidizes the PB, contained in the sensing electrode, which is subsequently reduced at the electrode and results in a detectable cathodic current signal. This decrease in the amperometric signal increases with increasing $H_2O_2$ concentration. For the compatibility with a standardized respiratory mask, the developed paper based $H_2O_2$ sensor is integrated into the housing of a commercially available airway filter mainly used in anaesthetic applications.

### Materials and Methods used in the Examples:

#### Chemical components and reagents

The chemicals and methods for the experiments are listed below. Unless otherwise stated, all chemicals were purchased from Sigma Aldrich, Germany.

o Humectants and electrolytes for sensor preparation

- 1 M potassium chloride (KCl)

- 0.1 M phosphate buffered saline (PBS) containing 0.1 M sodium chloride (NaCl)

- 10× PBS: 1.37 M NaCl, 27 mM KCl in 0.1 M PBS

o Hydrogen peroxide (30 wt%, Merck KGaA, Germany)

o 1 mM ferrocenemethanol for the electrochemical characterization of the paper based sensors

All electrochemical measurements in this work were performed with a potentiostat EmStat3 with an eight-channel multiplexer MUX8 and the corresponding software PSTrace 5.4 (PalmSens, The Netherlands).

#### Resolution of screen-printing

For assessing the limitation of screen printing, a mask with different structures for resolution testing was designed with CleWin (WieWeb software, The Netherlands) and ordered from Beta Layout GmbH (Germany). The test structures comprise lines with different widths (0.05 to 3 mm) and distances (0.05 to 1.5 mm), arrays of 3×3 circles with different diameters (0.05 to 3 mm) and squares of different edge lengths (0.05 to 3 mm), as illustrated in **Figure 4.** These structures were screen printed onto a paper substrate, by utilizing carbon paste purchased from Gwent Group (UK) and a squeegee. With this, the minimum width, realizable with this procedure, was determined. The smallest structures screen-printable were 100 μm thin lines, but their outcome was very inconsistent. In addition, 200 μm circles and squares, showed a uniform result, except that single lines of the array did not work for the 200 and 300 μm structures. These deficiencies might also stem from irregularities in the mask for such small structures. Therefore, structures with a width less than 300 $\mu$m were not considered further for electrode design.

*Resistance measurements*

To determine the minimum width with acceptable values for the conducting paths, resistance measurements were performed. As the voltage dependent current is gauged by amperometry, the resistance of the electrode structures has an impact on the sensor performance. Therefore, structures with different widths, as shown in **Figure 5,** were screen printed and their resistance was measured with the 4-point probes method.

Herein, carbon paste on paper and foil substrates, as well as the Prussian Blue (PB) and cobalt phthalocyanine (CP) mediated carbon pastes on paper were tested. In addition, the resistance of structures with silver / silver chloride beneath the carbon on paper were determined. The width of the measured structures ranged between 3 to 0.5 mm. The resulting resistances for structures with different materials and different widths are summarized in **Table 1.**

As expected, the resistance of the structures increases with increasing width due to:

$$R = \rho \cdot \frac{l}{A}$$

With the electrical resistivity $\rho$, the length of the conductor $l$ and the cross-sectional area $A$ (Marinescu, M. and Winter, J., Grundlagenwissen Elektrotechnik: Gleich-, Wechsel- und Drehstrom. Vieweg+Teubner Verlag, 2011), which in composed of the width and the height $h$ of the structure:

$$A = w \cdot h$$

Due to the high resistivity of the carbon pastes, the resistance was fairly high for the width of 1 mm preferred for the final chip design. Additional silver/silver chloride tracks were printed beneath the carbon tracks in order to decrease the resulting resistance. This width was chosen due to good results of the resolution test and as it offers an optimal size for a compact chip design.

*Evaluation of different electrode designs*

Two different electrode shapes, with the same 2D area, but different edge lengths, resulting in a different 3D area, were designed and fabricated. The idea was to assess the influence of the edge area on the current signal, as a larger overall area should lead to a higher signal. The two chip designs with differently shaped working electrodes are depicted in **Figure 6.** The bend electrode (design 2, **Figure 6B)** has a larger edge area, which is 1.55-times bigger than the round electrode (design 1, **Figure 6A).** Please note that the electrode height on the paper is presumed to be the same on a rigid substrate and taken from the product datasheet of the manufacturer.

To determine a suitable voltage for the amperometric signal readout, previously multi-step amperometry was performed in 0.1 M PBS and 35.28 $\mu$M $H_2O_2$ in the range between -0.2 and 0.9 V with 50 mV steps. These results are illustrated in **Figure 7A-C** and show that the carbon paste shows no reaction to $H_2O_2$ in a potential range from -0.1 to 0.45 V *versus* Ag/AgCl (0.1 M PBS). For PB, a voltage of 0 V and for CP, 0.4 V were chosen as at these potentials highest current signals for $H_2O_2$, compared to PBS, were obtained.

To compare the two designs, calibration curves of $H_2O_2$ were taken by means of amperometry using CP- and PB-mediated electrodes. Herein, the current signals for different $H_2O_2$ concentrations at a constant voltage of 0.0 V for PB and 0.4 V for CP were recorded. The CP paste proved to deliver lower current densities than the PB paste. The sensitivity for the CP paste was 0.053 and 0.041 nA $\mu$M$^{-1}$ mm$^{-2}$ with correlation coefficients of 0.99 for design 1 and 2, respectively. In the case of the PB mediated paste, the sensitivities were 0.12 and 0.16 nA $\mu$M$^{-1}$ mm$^{-2}$ with correlation coefficients of 0.99 for design 1 and 2, *vice versa.* The calibration curves with the resulting mean current densities are illustrated in **Figure 8.** In the case of PB, the curved structure of the working electrode results in higher current densities as the diffusion of the analyte is enhanced at the edges by using such an electrode design compared to the circular one. Surprisingly, this was not the case for the CP paste which might be caused by the inappropriate assumption of the electrode height on the paper substrate. For the final chip design, the PB-mediated carbon paste and the curved electrode (design 2) were chosen, since the obtained current densities for this combination delivered the highest signals.

*Differential electrode design*

The differential sensor design comprises two working electrodes on a single chip. One of these is the sensing electrode, containing PB as mediator and the other one consists of carbon paste without mediator serving as blank electrode to filter the background noise. Due to the similar resistance values of the carbon paste and the PB mediated paste, the current signal was expected to behave likewise and thus, signals coming from other sources than the oxidation of hydrogen peroxide could be easily excluded.

For the preferred paper based sensor with the differential electrode design, a calibration curve of $H_2O_2$, as illustrated in **Figure 9,** was carried out by amperometric measurements at a voltage of 0 V *versus* screen-printed Ag/AgCl. Here, $H_2O_2$ concentrations in a range between 5 and 160 $\mu$M delivered a linear current response with a sensitivity of 0.23 nA $\mu M^{-1}$ $mm^{-2}$ and a correlation coefficient of 0.99.

## *System integration*

To enable a comfortable use of the developed $H_2O_2$ sensor in on-site or clinical breath monitoring, it is beneficial to be compatible with a common respiratory mask. For this purpose, the housing of a commercially available filter for anaesthetic applications (Ultipor® 25, Pall corporation, US) was modified. Herein, the filter was removed from the housing and the sidewalls were replaced by customized 3D printed sidewalls. These sidewalls were designed with SolidWorks 2017 (Dassault Systèmes, France), so that the chip fits airtight into the housing and the contact pads of the sensor are located on the outside. They were manufactured via 3D printing with the Ultimaker 3 Extended (Geldermalsen, The Netherlands).

**Figure 10** shows an image of the respiratory mask with the extension containing the paper based $H_2O_2$ sensor. As paper itself is vulnerable, it is beneficial to stabilize the paper before the integration into the housing for the demonstrator version. Therefore, 1 mm thick poly(methyl methacrylate) (PMMA) sheets with a double sided adhesive film were lasered and the paper based sensors were placed in between two PMMA sheets. With these carriers, not only the stabilization of the paper based sensors is achieved, but also the conducting tracks are isolated against humidity, while an opening ensures that the electrodes are exposed to the breath.

## *Electrochemically active electrode area*

Due to the roughness of the paper substrate, the active surface area of the screen-printed electrodes could deviate largely from the geometric area. To examine the electrochemically active surface area, cyclic voltammograms of screen-printed carbon electrodes on both, paper and foil, were performed at different scan rates, between 25 and 200 mV $s^{-1}$. First, the capacitive contribution was determined in 50 mM KCl. Then, CVs in 1 mM ferrocenemethanol were recorded to identify the peak currents $I_p$ for the reduction peaks at the different scan rates $v$. After subtracting the capacitive current signals, the mean values of the peak currents (n = 4) were plotted over the square root of the scan rate and the slope was determined to calculate the electrochemically active electrode area $A$. The results are shown in **Figure 11.** The relation between these variables is described in the rearranged Randles-Sevcik equation:

$$A = \frac{I_p}{v^{\frac{1}{2}}} \cdot (2.69 \cdot 10^5 \cdot n^{\frac{3}{2}} \cdot D^{\frac{1}{2}} \cdot c_0)^{-1}$$

with the number of transferred electrons $n$, the diffusion coefficient $D$ of the electroactive species and the bulk concentration of the redox molecules $c_0$.

The ratio between the electrode areas on paper and foil was calculated by considering that the paper electrodes have a larger surface area. The assumption is that the foil blocks one whole side of the electrode and therefore, the area of the foil electrode only amounts 51% of the paper electrode area. However, taking this into account, the resulting experimental ratio of 0.959 implies, that the electrochemically active area on paper is insignificantly smaller than the one on foil. A possible reason might be that paper fibers block a part of the electrode surface and therewith, reduce its availability for the redox active species.

## *Study of different electrolytes*

Since the paper itself is not well-conductive, it is necessary to treat the paper with an electrolyte prior to an experiment. This was done by placing an electrolyte droplet onto the paper and allowing it to dry, before measuring in vapor. For the first measurements, 0.1 M PBS was used, but during the measurement the sensor got dry quickly and it was not possible to assure constant conditions. To overcome this problem, three different electrolytes were tested, regarding their sensing performance under dry and wet conditions. The used solutions include 1 M potassium chloride, 0.1 M PBS, 10× PBS. Each of these was applied to a sensor (100 $\mu$l) and left to dry for one day. Subsequently, CVs were recorded, first with the dry sensors, second the sensor was wetted with 50 $\mu$l of DI water and finally, a droplet of 200 $\mu$l 160 $\mu$M $H_2O_2$ was

added.

It turned out the tested solutions different in their capabilities to wet the paper or interfere with a hydrogen peroxide signal. CVs in 1 M KCl delivered better characteristics and results than 0.1 M PBS, as shown in **Figure 12.** Furthermore, the amperometric measurement of $H_2O_2$ in 1 M KCl provided a better sensitivity than in 0.1 M PBS. Therefore, 1 M KCl was chosen as electrolyte for further experiments.

*Stability of hydrogen peroxide solution*

For evaporating hydrogen peroxide, a commercially available humidifier HME-BOOSTER® (Medisize, The Netherlands), consisting of a heater element, an intake for injecting the solution and an outlet for the vapor, was employed as shown in **Figure 13.** During evaporation of hydrogen peroxide, diluted in a KCl solution, salt crystals formed, which blocked the pores of the humidifier. Therefore, from then on hydrogen peroxide was diluted in deionized water (DI water). This led to the problem, that lower current signals were observed. For this reason, the stability of $H_2O_2$ in DI water was studied and compared to $H_2O_2$ stability in 1 M KCl. Herein, amperometric measurements were performed with a fresh solution of $H_2O_2$ (10 minutes) and again after 90 minutes at 0 V **(Figure 13).** As the stability *de facto* was worse in DI water than in 1 M KCl, but potassium chloride was crystallized in the evaporator, the compromise was to prepare the stock solution with 1 M KCl and then dilute the stock solution with DI water to the desired concentration immediately before evaporating.

*Offset correction of measured current signals*

The measured current densities of blank and signal electrodes do not have the same baseline. This can be corrected by setting these signals to "zero" using an offset value prior to the calibration measurement.

*Correlation of vaporous and aqueous $H_2O_2$ measurement*

By division with a constant factor, a parallel linear plot **(Figure 3D)** can be obtained which is very close to the calibration curve in **Figure 2A.** Thus, the sensitivity of the sensor is reproduced under the measuring conditions in the water-saturated vapor. Therefore, it can be assumend that the paper sensor shows the same response with the same sensitivity as in solution. By adding a factor to the x values, the measured values in vapor can be superposed perfectly with the calibration curve. After this operation, the ratio between the original $H_2O_2$ concentrations of the prepared solutions used for the artificial breath and the obtained correlated concentrations are not completely conserved. For example, the ratio is 320:160 = 2 in aqueous, but only 42.91:22.73 = 1.89 in vapor. However, this may be caused by inaccuracies in the supply by the perfusor, and the evaporation would cause concentration fluctuations in the vapor.

*Measurement setup for exhaled breath analysis*

For the exhaled breath analysis, a measurement setup was installed and human respiration, as well as $H_2O_2$ containing breath were simulated. An image of this setup is illustrated in **Figure 16.**

**Example 1: Fabrication procedure for the electrochemical sensor**

The fabrication procedure for the paper sensors is illustrated schematically in **Figure 1A.** First, wax patterns are printed on chromatography paper (grade 1 CHR, 200×200 mm², Whatman, UK) using a commercially available wax printer (ColorQube 8580, Xerox corporation, US) and baked for 10 minutes at 120 °C in a conventional oven. When heated, the layer of wax printed on the surface of paper wicks through the bulk of the substrate and forms a hydrophobic barrier, defining the electrolytic cell. The wax barrier plays two important roles: i) It prevents wicking of any droplets of water condensed during exhalation to the contact pads during operation. ii) The wax pattern contains a solution of electrolyte before the water is evaporated from the substrate to form a solid-electrolyte. Next, the Ag/AgCl reference electrode (RE) and conducting tracks are screen-printed and baked for 10 minutes at 80 °C. Finally, the carbon counter (CE), blank and PB-mediated sensing electrodes are screen-printed and baked for 15 minutes at 80 °C.

The paper based sensor chip is placed inside a wearable respiratory mask, such that the patient is breathing directly onto the sensor. For integration into the ventilation mask, the paper chip is glued between two PMMA sheets with an opening for the electrodes, as depicted in **Figure 1B.** With this, the sensor is mechanically stabilized and, at the same time, the conducting tracks are isolated from potential shorts due to water droplets originating from exhaled breath. Furthermore, the housing of a commercial filter is modified **(Figure 1C)** by replacing the sidewalls with custom-made 3D printed parts to mount the paper based sensor into the housing which allows to place it directly in the respiratory flow. With this approach, moisture from the breath is captured by the paper sensor to humidify the paper substrate, forming an electrochemical cell, which is crucial for the operability of the sensor. The entire system is illustrated in **Figure 1D.**

Because paper itself is not ionically conductive, a droplet of electrolyte is placed on the paper and dried, before measuring analytes from exhaled breath. For the first measurements, 0.1 M phosphate buffered saline (PBS) is used as electrolyte, but during the measurement the sensor dries more quickly making it more difficult to maintain constant conditions. To solve this problem, three different electrolytes were tested (see **Figure 12).** Cyclic voltammograms (CVs) have been performed in dry (after one day) and wet (DI water added) condition and finally, with a droplet of 160 $\mu$M $H_2O_2$. The compounds tested exhibited difference in their ability to keep the paper wet and a possible interference with the detection of $H_2O_2$. According to the test results, 1 M potassium chloride (KCl) provides the best characteristics for the CVs and sensitivity for $H_2O_2$ in amperometric measurements. It has been, thus, chosen as an electrolyte salt for further experiments.

**Example 2: Calibration and amperometric measurements using the sensor**

For the calibration of the paper based $H_2O_2$ sensor, the current behaviour over time is recorded for different hydrogen peroxide concentrations. Amperometry at a constant potential of 0.0 V *versus* Ag/AgCl (screen-printed RE electrode) is carried out using different paper chips (n = 7). The frontside of the electrodes is isolated using an adhesive tape, as the paper sensors are positioned in the respiratory mask with the backside facing the user, hence, the frontside of the electrode structures has no direct contact with the exhaled breath. First, a droplet of 1 M KCl solution is placed on the electrolytic cell of the paper chip, and then, measurements with increasing the $H_2O_2$ concentration are performed. The obtained calibration curve is shown in **Figure 2A.** Here, a linear measurement range between 5 to 320 $\mu$M hydrogen peroxide is achieved with a sensitivity of 0.19 nA $\mu M^{-1}$ $mm^{-2}$ and a correlation coefficient of 0.99.

To mimic the human respiration, it is necessary to create a periodic air flow generating a warm and humid gas flow using a lung simulator, as the human exhaled breath contains ~100% RH at a temperature of around 34°C. Using a customized LabVIEW software (National Instruments, USA), the lung simulator pumps a desired volume of air with a predefined frequency. RH and temperature are adjusted using a commercially available humidifier (HumiCare® 200, Gründler Medical, Germany) that contains heated tubing. To introduce different concentrations of $H_2O_2$, an evaporator with a heating element is placed in between the lung simulator and the paper sensor. A scheme of this setup is illustrated in **Figure 2B.**

Since the moisture content of paper is varying with changing RH during inhalation and exhalation, to study the effect of RH on the redox characteristics of the PB-mediated carbon electrode, CV measurements using a dry chip, pre-treated with 1 M KCl, in $H_2O_2$-free simulated breath were performed. In all experiments (except the tests of respiration frequency and volume), the lung simulator is set to generate a tidal volume of 500 ml and a frequency of 15 breaths per minute, which are realistic values for a healthy adult. As it can be observed in **Figure 2C,** the initially dry sensor can be wetted only by the respiratory stream itself, assuring a high and more reliable electrochemical signal. After 195 breaths (13 minutes), the measured current signals do not alter anymore and exhibit a typical PB-CV of a wet sensor in 1 M KCl.

In **Figure 3A,** the current signal of an amperometric measurement at different respiration frequencies is illustrated. It is noticeable, that slower breathing results in a lower frequency of the signal measured and vice versa, while no significant signal change is observed by a volume change (see **Figure 3B).** During one breathing period, the water content in the paper changes periodically, as the air stream is drier during inhalation and reaches a RH of ~100% during exhaling. Accordingly, the ionic conductivity of the paper fibers changes[21,23]. Even though variations in conductivity are less important in an amperometric setup, the signal might decrease if the paper becomes too dry (see **Figure 2C).** However, as the blank electrodes without PB show a similar response, we conclude that the periodic variations must be mostly attributed to capacitive currents due to the humidity dependent changes of the dielectric properties of the paper[24]. Probably only fibers in direct contact with the electrode surface contribute to this effect. Hence, this capacitive part of the current is probably quite sensitive to the surface morphology of every individual electrode and is expected to reach its maximum at the reversal points of the respiratory movement.

In order to obtain a calibration curve for hydrogen peroxide in the vapor of the artificial breath, $H_2O_2$ solutions of different concentrations are evaporated and the current signal over time is recorded continuously. A typical measurement is shown in **Figure 3C.** As soon as a steady-state current is reached, the next higher concentration is added, as indicated with the arrows labelled with the corresponding concentration. The response time to obtain a steady-state current depends on the peroxide concentration in the vapor. The reason for this behaviour is probably due to the time required for the concentration of $H_2O_2$ in vapor to equilibrate with its dissolved form in water (i.e. dissolved in the moisture within paper). At higher $H_2O_2$ concentrations in the vapor, the gradient between vapor and "paper electrolyte" is higher and thus, a steeper current increase, as well as a higher limiting current are expected which is almost in line with our observations.

The behaviour of the blank (background) electrode can be also observed in **Figure 3A-C.** The measured blank signals do not settle at the same baseline currents as the sensing electrode. However, these different baseline currents can be aligned for the evaluation by setting an offset value (see **Figure 14).**

For the construction of the calibration curve, the current densities of the blank curve are first subtracted from those of the sensor electrode. After averaging and baseline subtraction, a measurement value is taken for each hydrogen peroxide concentration at a point on the timeline shortly before the next higher concentration is introduced. The baseline value of the sensor is taken right before addition of the first hydrogen peroxide concentration of 40 $\mu$M. The mean values for the calibration curve presented in **Figure 3D** are obtained from three independent measurements.

From these results, it can be concluded that $H_2O_2$ concentrations in the range between 40 and 320 $\mu$M give rise to a response with a sensitivity of 0.02 nA $\mu$M$^{-1}$ mm$^{-2}$ and a correlation coefficient of 0.99. It is crucial to note, however, that the resulting current signals for the respective $H_2O_2$ concentrations are significantly lower than of the former calibration in aqueous solutions **(Figure 2A)**. This may be due to the heating of the $H_2O_2$ in the evaporator and the poor stability of $H_2O_2$ in DI water (see **Figure 13**). By correlating the obtained current densities with those of the previous calibration in solution, the real $H_2O_2$ concentrations in the vapor of the artificial breath can be estimated to lie between 5 and 40 $\mu$M. This means that, after evaporation, the $H_2O_2$ concentration may be decreasing to approximately 1/8 of its original value while the same sensitivity as in solution is maintained, i.e. 0.19 nA $\mu$M$^{-1}$ mm$^{-2}$ **(Figure 3D and Figure 15)**. The humid air from the humidifier needs may also be diluting the analyte yielding a smaller concentration. Nevertheless, after accounting for all these factors, a reliable quantification of different hydrogen peroxide concentrations in vapor is achieved and, the proof-of-concept for on-site $H_2O_2$ analysis in exhaled breath is successfully demonstrated.

In summary, this example describes a differential electrochemical method using low-cost porous materials (for example, a low-cost cellulose paper) for on-site monitoring of hydrogen peroxide in exhaled breath. For compatibility with standardized ventilation masks, the sensor developed may be integrated into the housing of a commercially available airway filter for anaesthetic applications. Under realistic conditions by simulating human respiration with authentic lung volume and respiration rate, the proof-of-principle of the hydrogen peroxide measurements in exhaled breath are successfully shown for the first time. With further modifications and improvements, this sensor model can be employed in a large variety of applications, including clinical or wearable monitoring of exhaled breath.

As evident from the data described herein the claimed method and sensor have the following advantages: (i) Because of differential measurements, the influence of various interfering substances and/or environmental conditions (for example, temperature and humidity) are eliminated, hence, the system always produces reliable results. (ii) By changing or modifying and/or coating the material of the substrate or the sensing electrode (for instance, with metals, metal oxide- or semiconducting micro- and nanoparticles, enzymes, selective membranes or conducting polymers), the sensor model presented can be extended for the analysis of other compounds from exhaled breath. (iii) A flexible and hygroscopic porous support, like paper, acts as a "solid electrolyte" eliminating the need for additional membranes (containing the electrolyte) and at the same time as a substrate for the electrodes. (iv) Flexible and porous substrates can be shaped and patterned in a way that the sensing surface as well as the collection volume can be considerably increased. (v) The orientation and porosity of the sensing surface can be tuned to minimize breathing resistance and to improve signal quality (i.e. signal-to-noise ratio).

The performance of this method and sensor can be further enhanced by: (i) screening for further humectants as possible electrolytes to facilitate the handling and signal processing, for example, by keeping the porous substrate wet and ensuring that the sensor does not need to adsorb any humidity from the breath. (ii) PB-mediated carbon paste with different PB contents and modification procedures may be further tested in order to further increase the $H_2O_2$ sensitivity. Alternatively, hydrophilic metal electrodes (especially Pt), realized by metallization of fabrics, may be employed[25]. Moreover, the implemented sensor system may be extended with a compact and low-power wearable signal readout unit along with a smartphone app to enable on-site monitoring.

**Example 3: Design and proof-of-principle for a paper-based glucose sensor**

*Chip design and fabrication for a paper-based glucose sensor*

The design and fabrication procedure for the paper-based glucose sensors were carried out according the methods described above in relation a paper-based hydrogen peroxide sensor and shown schematically in **Figure 16.**

The only difference in the chip design is the use of two identical compartments, separated with wax, but still employing a common reference and a counter electrode. The fabrication starts with printing wax patterns on the chromatography paper (grade 1 CHR, $200 \times 200$ mm$^2$, Whatman, U.K.) by means of a commercial wax printer (ColorQube 8580, Xerox corporation, USA). This is followed by a 10-min bake at 120 °C in an oven which results in the wicking of wax printed through the paper substrate and thus, defines a hydrophilic area for the electrolytic cell. At the next step, the reference electrode (RE) and conducting tracks are screen-printed with silver/silver chloride (Ag/AgCl) paste (C2040308P2, Gwent Group, U.K.) and baked for 10 min at 80 °C. Last, the carbon counter (CE) and PB-mediated working electrodes are screen-printed using the carbon and mediated carbon pastes (C2030519P4 and C2070424P2, Gwent Group, U.K.).

*On-paper functionalization of glucose oxidase*

Glucose oxidase (GOx) solved in 1 M potassium chloride (KCl) is adsorbed into the compartment surrounding the sensing electrode. Alternatively, GOx can be either immobilized covalently (for example, by using glutaraldehyde), encapsulated (by polyethylenimine), or entrapped in a gel (such as hydrogel) into the paper substrate[32].

GOx catalyses the oxidation of glucose into hydrogen peroxide ($H_2O_2$) which can be reduced at the screen-printed Prussian Blue (PB)-mediated carbon electrode. The measured current relates directly to the glucose concentration of the sample. A second, identical cell, but only treated with 1 M KCl (without GOx), enables to subtract background signals and periodic variations caused by the respiratory movement.

*Results of a proof of principle study for the non-invasive glucose sensing approach*

To demonstrate the proof-of-principle of the described non-invasive glucose sensing approach, the sensor is exposed to aerosols of different glucose concentrations (5 $\mu$M to 10 mM) using a deodorant nebulizer. Within the physiological range, a stepwise increase of the differential current signals (see **Figure 18**) at consecutive aerosol administrations is observed. At higher concentrations, a peak followed by a current decay is noticed, possibly due to limited oxygen supply and thus, a limitation of enzyme activity.

Glucose entrapped in aerosols can be cumulatively sampled and directly measured with paper-based sensors at concentrations of less than 5 $\mu$M. Compared to EBC analysis, our approach minimizes the risk of analyte degradation, while considerably reducing acquisition time and system's complexity. It also allows continuous glucose monitoring.

As illustrated in **Figure 19** and **20** advantageously the paper-based sensor may be integrated into a conventional respiratory mask in a straightforward manner. To this end the paper-based sensor can be easily applied directly onto the mask, e.g. using by isolating it with a flexible tape. Via a suitable chip connector and wires the sensor may be connected to a potentiostat, which is connected to a mobile device such as a smart phone.

In conclusion a proof-of-principle of a facile, inexpensive and non-invasive approach for the simultaneous sampling and measurement of exhaled glucose could be demonstrated, for the first time. Further improvement may include the characterization and optimization of the developed system in simulated breath, followed by a further clinical validation.

**Table 1.** Mean values of resistances for screen-printed structures with different widths and materials.

| | Mean value of resistance in $\Omega$ | | | | | | | |
|---|---|---|---|---|---|---|---|---|
| Width in mm | 3 | 2.5 | 2 | 1.5 | 1 | 0.9 | 0.2 | |
| Carbon on paper | 261.2 ± 4 5 | 296.8 ± 55 | 385.8 ± 5 2 | 477.4 ± 56 | 765.2 ± 12 7 | 843.2 ± 10 8 | 1477.0 ± 28 5 | n = 5 |
| Carbon on foil | 228.3 ± 6 | 276.7 ± 16 | 375.7 ± 4 2 | 475.0 ± 58 | 682.0 ± 60 | 769.0 ± 83 | 1372.0 ± 48 | n = 3 |
| PB mediate d carbon on paper | 294.3 ± 4 6 | 313.7 ± 29 | 358.0 ± 1 7 | 465.7 ± 28 | 691.0 ± 35 | 820.0 ± 13 5 | 1345.0 ± 75 | n = 3 |
| CP mediate d carbon on paper | 242.7 ± 2 3 | 267.7 ± 6 | 306.3 ± 1 1 | 420.3 ± 32 | 662.7 ± 52 | 818.3 ± 14 7 | 1414.3 ± 32 0 | n = 3 |
| Ag/AgCl tracks beneath carbon on paper | 0.07 ± 0 | 0.08 ± 0.0 1 | 0.1 ± 0 | 0.12 ± 0.0 2 | 0.19 ± 0.02 | 0.20 ± 0.01 | 0.50 ± 0.06 | n = 3 |

**REFERENCES**

1. World Health Organization. WHO releases country estimates on air pollution exposure and health impact. (2016).

2. World Health Organization. The Global Impact of Respiratory Disease. (2017).

3. World Health Organization. Chronic respiratory diseases. (2004). Available at: https://www.who.int/respiratory/en/. (Accessed: 23rd August 2018)

4. World Health Organization. Fact sheet - Noncommunicable diseases. (2018). Available at: www.who.int/news-room/fact-sheets/detail/noncommunicable-diseases. (Accessed: 23rd August 2018)

5. Zhou, M., Liu, Y. & Duan, Y. Breath biomarkers in diagnosis of pulmonary diseases. Clin. Chim. Acta 413, 1770-1780 (2012).

6. Svensson, S., Olin, A., Larstad, M., Ljungkvist, G. & Toren, K. Determination of hydrogen peroxide in exhaled breath condensate by flow injection analysis with fluorescence detection. J. Chromatogr. B 809, 199-203 (2004).

7. Paget-Brown, A. O. et al. Normative Data for pH of Exhaled Breath Condensate. Chest 129, 426-430 (2006).

8. Solvay Chemicals Inc. Hydrogen peroxide safety and handling, technical datasheet. (2015). Available at: https://www.solvay.us/en/binaries/HH-2323-236798.pdf. (Accessed: 21st August 2018)

9. Horváth, I., Hunt, J. & Barnes, P. J. Exhaled breath condensate: methodological recommendations and unresolved questions. Eur. Respir. J. 26, 523-548 (2005).

10. Gerritsen, W. B. M., Asin, J., Zanen, P., Bosch, J. M. M. va. den & Haas, F. J. L. M. Markers of inflammation and oxidative stress in exacerbated chronic obstructive pulmonary disease patients. Respir. Med. 99, 84-90 (2005).

11. Dohlman, A. W., Black, H. R. & Royall, J. A. Expired Breath Hydrogen Peroxide Is a Marker of Acute Airway Inflammation in Pediatric Patients with Asthma. Am. Rev. Respir. Dis. 148, 955-960 (1993).

12. Komkova, M. A., Karyakina, E. E., Marken, F. & Karyakin, A. A. Hydrogen Peroxide Detection in Wet Air with a Prussian Blue Based Solid Salt Bridged Three Electrode System. Anal. Chem. 85, 2574-2577 (2013).

13. Mutlu, G. M., Garey, K. W., Robbins, R. A., Danziger, L. H. & Rubinstein, I. Collection and analysis of exhaled breath condensate in humans. Am. J. Respir. Crit. Care Med. 164, 731-7 (2001).

14. Dincer, C. et al. Disposable Sensors in Diagnostics, Food, and Environmental Monitoring. Adv. Mater. 1806739 (2019). doi:10.1002/adma.201806739

15. Bandodkar, A. J., Jeerapan, I. & Wang, J. Wearable Chemical Sensors: Present Challenges and Future Prospects. ACS Sensors 1, 464-482 (2016).

16. Kenry, Yeo, J. C. & Lim, C. T. Emerging flexible and wearable physical sensing platforms for healthcare and biomedical applications. Microsystems Nanoeng. 2, 16043 (2016).

17. Koydemir, H. C. & Ozcan, A. Wearable and Implantable Sensors for Biomedical Applications. Annu. Rev. Anal. Chem. 11, (2018).

18. Yang, Y. & Gao, W. Wearable and flexible electronics for continuous molecular monitoring. Chem. Soc. Rev. 48, 1465-1491 (2019).

19. Rolland, J. P. & Mourey, D. A. Paper as a novel material platform for devices. MRS Bull. 38, 299-305 (2013).

20. Dincer, C., Bruch, R., Kling, A., Dittrich, P. S. & Urban, G. A. Multiplexed Point-of-Care Testing - xPOCT. Trends Biotechnol. 35, 728-742 (2017).

21. Barandun, G. et al. Cellulose fibers enable near zero-cost electrical sensing of water-soluble gases. ACS Sensors acssensors.9b00555 (2019). doi:10.1021/acssensors.9b00555

22. Bracher, P. J., Gupta, M. & Whitesides, G. M. Patterning precipitates of reactions in paper. J. Mater. Chem. 20, 5117 (2010).

23. Güder, F. et al. Paper-Based Electrical Respiration Sensor. Angew. Chemie Int. Ed. 55, 5727-5732 (2016).

24. Gaspar, C., Olkkonen, J., Passoja, S. & Smolander, M. Paper as Active Layer in Inkjet-Printed Capacitive Humidity Sensors. Sensors 17, 1464 (2017).

25. Grell, M. et al. Autocatalytic Metallization of Fabrics Using Si Ink, for Biosensors, Batteries and Energy Harvesting. Adv. Funct. Mater. 29, 1804798 (2019).

26. WHO. Diabetes. 2020 https://www.who.int/health-topics/diabetes.

27. Choi, H. Recent developments in minimally and truly non-invasive blood glucose monitoring techniques. in 2017 IEEE SENSORS 1-3 (IEEE, 2017). doi:10.1109/ICSENS.2017.8234291.

28. Dincer, C. et al. Disposable Sensors in Diagnostics, Food, and Environmental Monitoring. Adv. Mater. 31, 1806739 (2019).

29. Kovalaske, M. A. & Gandhi, G. Y. Glycemic Control in the Medical Intensive Care Unit. J. Diabetes Sci. Technol. 3, 1330-1341 (2009).

30. Tankasala, D. & Linnes, J. C. Noninvasive glucose detection in exhaled breath condensate. Transl. Res. 213, 1-22 (2019).

31. Ferrante do Amaral, C. E. & Wolf, B. Current development in non-invasive glucose monitoring. Med. Eng. Phys. 30, 541-549 (2008).

32. Nery, E. W. & Kubota, L. T. Evaluation of enzyme immobilization methods for paper-based devices-A glucose oxidase study. J. Pharm. Biomed. Anal. 117, 551-559 (2016).

33. Ghoresihizadeh Sara S. "An integrated Platform for diffrential electrochemical and ISFET sensing, IEEE International Symposium on circuits and systems (ISCAS) (2016).

34. Güder Firat et al. "Paper-Based Electrical Respiration Sensor, Angewandte Chemie Internationale Edition, vol. 55, p. 5727-5732 (2016).

## Claims

1. Electrochemical sensor for monitoring the presence of an analyte in the breath of a subject, comprising

    i. a support comprising a porous substrate material
    ii. at least one pair of working electrodes as well as at least one counter and/or reference electrode, which are at least partially integrated into said porous supporting material
    the at least two working electrodes comprise an analyte-sensitive sensing electrode and an analyte-insensitive blank electrode and wherein a salt is immobilized in said support, such that upon exhaling onto the sensor a differential electrochemical measurement at said pair of working electrodes allows for monitoring the presence of the analyte in the breath of said subject and
    wherein the support is air-permeable such that the breath may at least partially flow through the electrochemical sensor and hygroscopic such that a liquid portion of the breath is captured to allow for dissolution of the salt immobilised in said support and
    wherein the salt immobilized in said support is hydrophilic to an extent that the humidity of human exhaled breath is sufficient to form a conductive electrolyte, and the salt immobilized in said support is hygroscopic to an extent sufficient to keep the porous substrate material wet.

2. Electrochemical sensor according to the previous claim
    **characterized in that**
    the support is flexible and/or wherein the support is selected from the group of porous membranes, consisting of a cellulose based material (such as paper), a ceramic, a hydrogel and/or a hydrophilic polymer.

3. Electrochemical sensor according to any one of the preceding claims
    **characterized in that** the analyte is selected from the group consisting of hydrogen peroxide, glucose, lactate, proteins, pathogens, genetic materials, hormones, hydrocarbons, aldehydes, sulfides, ammonia, ethanol, acetone, isoprene, ethane, carbonyl sulfides, carbon dioxides, carbon monoxide, nitrogen monoxide and volatile organic compounds (VOCs).

4. Electrochemical sensor according to any one of the preceding claims
   **characterized in that**

   the sensing electrode or porous substrate comprises an analyte-sensitive material, which is a catalyst for an electrochemical reaction of the analyte,
   the sensing electrode comprises an analyte-sensitive receptor (for example, antibodies, binding proteins, aptamers, DNAs, RNAs or molecularly imprinted polymers), which causes an electrically measurable signal change (such as impedance or conductance) in dependence of the analyte concentration
   and/or the material of the sensing electrode is supplemented with and/or coated with an analyte-sensitive material, preferably selected from the group consisting of metal, metal oxide or semiconducting micro- or nanoparticles, enzymes, selective membranes and conductive polymers.

5. Electrochemical sensor according to any one of the preceding claims
   **characterized in that**
   the analyte is hydrogen peroxide and the sensing electrode consists of a metal, e.g., platinum, or comprises metal micro/nanoparticles or a mediator, e.g., Prussian Blue or Cobalt Phthalocyanine, as an analyte-sensitive material.

6. Electrochemical sensor according to any one of the preceding claims
   **characterized in that**

   the salt immobilized in said support is selected from the group consisting of potassium chloride, sodium chloride, sodium acetate, ammonium acetate, monosodium phosphate and buffer a salt mixture, e.g. phosphate buffer, most preferably the salt is potassium chloride and/or
   the salt is immobilized in the support by applying a solution containing the salt on the porous material and letting said solution dry before the use of the sensor.

7. Electrochemical sensor according to any one of the preceding claims
   **characterized in that**

   the electrochemical sensor comprises at least one pair of working electrodes, at least one counter electrode and optionally, one reference electrode,
   and/or
   the electrochemical sensor comprises at least two or more pairs of working (preferably geometrically identical) electrodes targeted at the detection of one or more, preferably two or more analytes.

8. Electrochemical sensor according to any one of the preceding claims
   **characterized in that**

   the at least two working electrodes are carbon, platinum, gold or silver electrodes, preferably producible by printing a suitable paste, e.g., a carbon, platinum, gold or silver paste, onto and/or into the support
   and/or
   the electrochemical sensor comprises a silver/silver chloride reference electrode and/or a preferably a carbon counter electrode.

9. Electrochemical sensor according to any one of the preceding claims
   **characterized in that**

   a structured pattern of a hydrophobic material, preferably a wax, is applied, preferably printed, onto the porous support material, thereby preferably forming an impermeable barrier inside the porous substrate material after final processing (for example, by baking), wherein preferably the structured pattern comprises an opening forming an electrochemical cell in which the at least one pair of working electrodes and the at least one reference electrode and/or counter electrode are located, and/or
   wherein the structured pattern may include different compartments inside the electrochemical cell in which the support and/or an electrode are sensitized for the analyte by coating and/or functionalization, wherein preferably an ion flow between the compartments is possible.

10. Electrochemical sensor according to any one of the preceding claims
    **characterized in that**

the electrochemical sensor additionally comprises a processing unit configured for the reading of electrically measurable signals, e.g., amperometric or impedimetric signals, of said electrodes and processing of said signals to monitor the presence of the analyte and/or the electrochemical sensor additionally comprises a communication interface for receiving and/or transmitting data to a mobile device.

11. A breath analysis and/or monitoring system comprising a) an electrochemical sensor according to any one of the preceding claims and b) a filter extension and/or c) a respiratory mask, wherein the electrochemical sensor is compatible with the filter extension and/or c) respiratory mask.

12. A method for an on-site (preferably wearable) or clinical monitoring of the presence of an analyte in the breath of a subject comprising

   a. Providing an electrochemical sensor according to any one of claims 1 - 10 or a breath analysis and/or monitoring system according to the previous claim 11
   b. Positioning said electrochemical sensor in the respiratory flow of said subject
   c. Employing a differential measurement by detecting the differential electrochemical signal at the at least one pair of working electrodes in order to monitor the presence of the analyte.

13. A method according to the previous claim
    **characterized in that**
    the presence of the analyte is monitored continuously during a single or multiple exhaling and inhaling cycles and/or wherein different segments of the monitored signal are used in order to quantify the presence of the analyte in different regions of a lung and/or airways.

14. A method according to any one of the preceding claims 12 or 13
    **characterized in that**
    the signal detected at the analyte-insensitive blank electrode is used for a background correction of non-specific interferences of the signal detected at said analyte-sensitive blank electrode, wherein preferably the background correction method is able to compensate current variations caused by the respiratory movement and environmental conditions.

**Patentansprüche**

1. Elektrochemischer Sensor zur Überwachung des Vorhandenseins eines Analyten im Atem eines Subjekts, umfassend

   i. einen Träger, umfassend ein poröses Substratmaterial
   ii. mindestens ein Paar Arbeitselektroden sowie mindestens eine Gegen- und/oder Referenzelektrode, die mindestens teilweise in das poröse Trägermaterial integriert sind
   wobei die mindestens zwei Arbeitselektroden eine analytempfindliche Messelektrode und eine analytunempfindliche Blindelektrode umfassen und wobei ein Salz in dem Träger derart immobilisiert ist, dass beim Ausatmen auf den Sensor eine differentielle elektrochemische Messung an dem Paar von Arbeitselektroden die Überwachung des Vorhandenseins des Analyten in der Atemluft des Subjekts ermöglicht und
   wobei der Träger luftdurchlässig ist, derart, dass der Atem mindestens teilweise durch den elektrochemischen Sensor strömen kann, und hygroskopisch ist, derart, dass ein flüssiger Abschnitt des Atems aufgenommen wird, um die Auflösung des in dem Träger immobilisierten Salzes zu ermöglichen, und
   wobei das in dem Träger immobilisierte Salz in einem solchen Maße hydrophil ist, dass die Feuchtigkeit der menschlichen Ausatemluft ausreicht, um einen leitfähigen Elektrolyten zu bilden, und das in dem Träger immobilisierte Salz in einem solchen Maße hygroskopisch ist, dass das poröse Substratmaterial feucht gehalten wird.

2. Elektrochemischer Sensor nach dem vorhergehenden Anspruch **dadurch gekennzeichnet, dass**
   der Träger flexibel ist und/oder wobei der Träger aus der Gruppe poröser Membranen ausgewählt ist, bestehend aus einem cellulosebasierten Material (wie beispielsweise Papier), einer Keramik, einem Hydrogel und/oder einem hydrophilen Polymer.

3. Elektrochemischer Sensor nach einem der vorhergehenden Ansprüche

**dadurch gekennzeichnet, dass** der Analyt aus der Gruppe bestehend aus Wasserstoffperoxid, Glukose, Laktat, Proteinen, Pathogenen, genetischem Material, Hormonen, Kohlenwasserstoffen, Aldehyden, Sulfiden, Ammoniak, Ethanol, Aceton, Isopren, Ethan, Carbonylsulfiden, Kohlendioxid, Kohlenmonoxid, Stickstoffmonoxid und flüchtigen organischen Verbindungen (VOCs) ausgewählt ist.

4. Elektrochemischer Sensor nach einem der vorhergehenden Ansprüche
**dadurch gekennzeichnet, dass**

die Sensorelektrode oder das poröse Substrat ein analytempfindliches Material umfasst, das ein Katalysator für eine elektrochemische Reaktion des Analyten ist,
die Sensorelektrode einen analytempfindlichen Rezeptor (zum Beispiel Antikörper, Bindungsproteine, Aptamere, DNAs, RNAs oder molekular geprägte Polymere) umfasst, der in Abhängigkeit von der Analytkonzentration eine elektrisch messbare Veränderung (wie beispielsweise Impedanz oder Leitfähigkeit) verursacht und/oder das Material der Sensorelektrode mit einem analytempfindlichen Material ergänzt und/oder beschichtet ist, das vorzugsweise aus der Gruppe ausgewählt ist, die aus Metall, Metalloxid oder halbleitenden Mikro- oder Nanopartikeln, Enzymen, selektiven Membranen und leitfähigen Polymeren besteht.

5. Elektrochemischer Sensor nach einem der vorhergehenden Ansprüche
**dadurch gekennzeichnet, dass**
der Analyt Wasserstoffperoxid ist und die Sensorelektrode aus einem Metall, z. B. Platin, besteht oder Metall-Mikro-/Nanopartikel oder einen Mediator, z. B. Preußischblau oder Kobaltphthalocyanin, als analytempfindliches Material umfasst.

6. Elektrochemischer Sensor nach einem der vorhergehenden Ansprüche
**dadurch gekennzeichnet, dass**

das in dem Träger immobilisierte Salz ausgewählt ist aus der Gruppe bestehend aus Kaliumchlorid, Natriumchlorid, Natriumacetat, Ammoniumacetat, Mononatriumphosphat und einer Puffersalzmischung, z. B. einem Phosphatpuffer, wobei das Salz vorzugsweise Kaliumchlorid ist und/oder
das Salz in dem Träger immobilisiert ist, indem eine Lösung, die das Salz enthält, auf das poröse Material aufgebracht wird und die Lösung vor der Verwendung des Sensors trocknen gelassen wird.

7. Elektrochemischer Sensor nach einem der vorhergehenden Ansprüche
**dadurch gekennzeichnet, dass**

der elektrochemische Sensor mindestens ein Paar Arbeitselektroden, mindestens eine Gegenelektrode und gegebenenfalls eine Referenzelektrode umfasst,
und/oder
der elektrochemische Sensor mindestens zwei oder mehr Paare von Arbeitselektroden (vorzugsweise geometrisch identisch) umfasst, die auf die Erkennung von einem oder mehreren, vorzugsweise zwei oder mehreren Analyten abzielen.

8. Elektrochemischer Sensor nach einem der vorhergehenden Ansprüche
**dadurch gekennzeichnet, dass**

die mindestens zwei Arbeitselektroden Kohlenstoff-, Platin-, Gold- oder Silberelektroden sind, die vorzugsweise durch Aufdrucken einer geeigneten Paste, z. B. einer Kohlenstoff-, Platin-, Gold- oder Silberpaste, auf und/oder in den Träger herstellbar sind
und/oder
der elektrochemische Sensor eine Silber/Silberchlorid-Referenzelektrode und/oder vorzugsweise eine Kohlenstoff-Gegenelektrode umfasst.

9. Elektrochemischer Sensor nach einem der vorhergehenden Ansprüche
**dadurch gekennzeichnet, dass**

ein strukturiertes Muster aus einem hydrophoben Material, vorzugsweise Wachs, auf das poröse Trägermaterial aufgebracht, vorzugsweise aufgedruckt ist, wodurch vorzugsweise nach der endgültigen Verarbeitung (zum Beispiel durch Einbrennen) eine undurchlässige Barriere innerhalb des porösen Substratmaterials gebildet wird, wobei das strukturierte Muster vorzugsweise eine Öffnung umfasst, die eine elektrochemische Zelle bildet, in der sich das mindestens eine Paar Arbeitselektroden und die mindestens eine Referenzelektrode und/oder Gegenelektrode befinden, und/oder

wobei das strukturierte Muster verschiedene Kompartimente innerhalb der elektrochemischen Zelle beinhalten kann, in denen der Träger und/oder eine Elektrode durch Beschichtung und/oder Funktionalisierung für den Analyten sensibilisiert sind, wobei vorzugsweise ein Ionenfluss zwischen den Kompartimenten möglich ist.

**10.** Elektrochemischer Sensor nach einem der vorhergehenden Ansprüche
**dadurch gekennzeichnet, dass**
der elektrochemische Sensor zusätzlich eine Verarbeitungseinheit umfasst, die zum Lesen elektrisch messbarer Signale, z. B. amperometrischer oder impedimetrischer Signale, der Elektroden und zum Verarbeiten dieser Signale konfiguriert ist, um das Vorhandensein des Analyten zu überwachen, und/oder der elektrochemische Sensor zusätzlich eine Kommunikationsschnittstelle zum Empfangen und/oder Senden von Daten an eine mobile Vorrichtung umfasst.

**11.** Atemanalyse- und/oder Überwachungssystem, umfassend a) einen elektrochemischen Sensor nach einem der vorhergehenden Ansprüche und b) eine Filterverlängerung und/oder c) eine Atemmaske, wobei der elektrochemische Sensor mit der Filterverlängerung und/oder c) der Atemmaske kompatibel ist.

**12.** Verfahren zur Vor-Ort-Überwachung (vorzugsweise mittels eines tragbaren Geräts) oder klinischen Überwachung des Vorhandenseins eines Analyten in der Atemluft eines Subjekts, umfassend

a. Bereitstellen eines elektrochemischen Sensors nach einem der Ansprüche 1 bis 10 oder eines Atemanalysesystems und/oder Überwachungssystems nach dem vorherigen Anspruch 11
b. Positionieren des elektrochemischen Sensors im Atemfluss des Subjekts
c. Anwenden einer Differenzmessung durch Erkennen des elektrochemischen Differenzsignals an dem mindestens einen Paar Arbeitselektroden, um das Vorhandensein des Analyten zu überwachen.

**13.** Verfahren nach dem vorhergehenden Anspruch
**dadurch gekennzeichnet, dass**
das Vorhandensein des Analyten während eines oder mehrerer Ausatmungs- und Einatmungszyklen kontinuierlich überwacht wird und/oder wobei verschiedene Segmente des überwachten Signals verwendet werden, um das Vorhandensein des Analyten in verschiedenen Bereichen einer Lunge und/oder Atemwegen zu quantifizieren.

**14.** Verfahren nach einem der vorhergehenden Ansprüche 12 oder 13
**dadurch gekennzeichnet, dass**
das an der analytunempfindlichen Blindelektrode erkannte Signal für eine Hintergrundkorrektur unspezifischer Störungen des an der analytempfindlichen Blindelektrode erkannten Signals verwendet wird, wobei das Hintergrundkorrekturverfahren vorzugsweise in der Lage ist, durch Atembewegungen und Umgebungsbedingungen verursachte Stromschwankungen auszugleichen.

**Revendications**

**1.** Capteur électrochimique pour la surveillance de la présence d'un analyte dans l'air expiré d'un sujet, comprenant

i. un support comprenant un matériau de substrat poreux
ii. au moins une paire d'électrodes de travail ainsi qu'au moins une contre-électrode et/ou une électrode de référence, qui sont au moins partiellement intégrées dans ledit matériau de support poreux
les au moins deux électrodes de travail comprennent une électrode de détection sensible à l'analyte et une électrode témoin insensible à l'analyte, et dans lequel un sel est immobilisé sur ledit support, de sorte que, lors de l'expiration sur le capteur, une mesure électrochimique différentielle au niveau de ladite paire d'électrodes de travail permet de surveiller la présence de l'analyte dans l'air expiré dudit sujet et
dans lequel le support est perméable à l'air de sorte que l'air expiré peut au moins partiellement traverser le capteur électrochimique et hygroscopique de sorte qu'une partie liquide de l'air expiré est capturée pour permettre la dissolution du sel immobilisé dans ledit support et
dans lequel le sel immobilisé dans ledit support est hydrophile dans une mesure où l'humidité de l'air expiré par l'homme est suffisante pour former un électrolyte conducteur, et le sel immobilisé dans ledit support est hygroscopique dans une mesure suffisante pour maintenir le matériau de substrat poreux humide.

2. Capteur électrochimique selon la revendication précédente **caractérisé en ce que**
le support est flexible et/ou dans lequel le support est choisi parmi le groupe de membranes poreuses, constitué d'un matériau à base de cellulose (tel que du papier), d'une céramique, d'un hydrogel et/ou d'un polymère hydrophile.

3. Capteur électrochimique selon l'une quelconque des revendications précédentes **caractérisé en ce que** l'analyte est choisi parmi le groupe constitué de peroxyde d'hydrogène, de glucose, de lactate, de protéines, d'agents pathogènes, de matériel génétique, d'hormones, d'hydrocarbures, d'aldéhydes, de sulfures, d'ammoniac, d'éthanol, d'acétone, d'isoprène, d'éthane, de sulfures de carbonyle, de dioxyde de carbone, de monoxyde de carbone, de monoxyde d'azote et de composés organiques volatils (COV).

4. Capteur électrochimique selon l'une quelconque des revendications précédentes **caractérisé en ce que**

l'électrode de détection ou le substrat poreux comprend un matériau sensible à l'analyte, qui est un catalyseur pour une réaction électrochimique de l'analyte,
l'électrode de détection comprend un récepteur sensible à l'analyte (par exemple, des anticorps, des protéines de liaison, des aptamères, des ADN, des ARN ou des polymères à empreinte moléculaire), qui provoque un changement de signal mesurable électriquement (tel que l'impédance ou la conductance) en fonction de la concentration de l'analyte
et/ou le matériau de l'électrode de détection est complété et/ou recouvert d'un matériau sensible à l'analyte, de préférence choisi parmi le groupe constitué de micro/nanoparticules métalliques, d'oxyde métallique ou semi-conductrices, d'enzymes, de membranes sélectives et de polymères conducteurs.

5. Capteur électrochimique selon l'une quelconque des revendications précédentes **caractérisé en ce que**
l'analyte est le peroxyde d'hydrogène et l'électrode de détection est constituée d'un métal, par exemple du platine, ou comprend des micro/nanoparticules métalliques ou un médiateur, par exemple du bleu de Prusse ou de la phtalocyanine de cobalt, en tant que matériau sensible à l'analyte.

6. Capteur électrochimique selon l'une quelconque des revendications précédentes **caractérisé en ce que**

le sel immobilisé dans ledit support est choisi parmi le groupe constitué du chlorure de potassium, du chlorure de sodium, de l'acétate de sodium, de l'acétate d'ammonium, du phosphate monosodique et d'un mélange de sels tampon, par exemple un tampon phosphate ; de préférence, le sel est le chlorure de potassium et/ou
le sel est immobilisé dans le support en appliquant une solution contenant le sel sur le matériau poreux et en laissant sécher ladite solution avant l'utilisation du capteur.

7. Capteur électrochimique selon l'une quelconque des revendications précédentes **caractérisé en ce que**

le capteur électrochimique comprend au moins une paire d'électrodes de travail, au moins une contre-électrode et éventuellement une électrode de référence,
et/ou
le capteur électrochimique comprend au moins deux paires ou plus d'électrodes de travail (de préférence géométriquement identiques) destinées à la détection d'un ou de plusieurs analytes, de préférence deux ou plus.

8. Capteur électrochimique selon l'une quelconque des revendications précédentes **caractérisé en ce que**

les au moins deux électrodes de travail sont des électrodes en carbone, platine, or ou argent, de préférence pouvant être produites par impression d'une pâte appropriée, par exemple une pâte de carbone, de platine, d'or ou d'argent, sur et/ou dans le support
et/ou
le capteur électrochimique comprend une électrode de référence en argent/chlorure d'argent et/ou de préférence une contre-électrode en carbone.

9. Capteur électrochimique selon l'une quelconque des revendications précédentes **caractérisé en ce que**

un motif structuré d'un matériau hydrophobe, de préférence une cire, est appliqué, de préférence imprimé, sur le matériau de support poreux, formant ainsi de préférence une barrière imperméable à l'intérieur du matériau de substrat poreux après traitement final (par exemple, par cuisson), dans lequel de préférence le motif structuré comprend une ouverture formant une cellule électrochimique dans laquelle sont situées l'au moins une paire d'électrodes de travail et l'au moins une électrode de référence et/ou une contre-électrode, et/ou dans lequel le motif structuré peut comporter différents compartiments à l'intérieur de la cellule électrochimique, dans lesquels le support et/ou une électrode sont sensibilisés à l'analyte par revêtement et/ou fonctionnalisation, dans lequel de préférence un flux d'ions entre les compartiments est possible.

10. Capteur électrochimique selon l'une quelconque des revendications précédentes **caractérisé en ce que**

le capteur électrochimique comprend également une unité de traitement configurée pour la lecture de signaux mesurables électriquement, par exemple des signaux ampérométriques ou impédimétriques, desdites électrodes et le traitement desdits signaux pour surveiller la présence de l'analyte et/ou le capteur électrochimique comprend également une interface de communication pour la réception et/ou la transmission de données à un dispositif mobile.

11. Système d'analyse et/ou de surveillance de l'air expiré comprenant a) un capteur électrochimique selon l'une quelconque des revendications précédentes et b) une extension de filtre et/ou c) un masque respiratoire, dans lequel le capteur électrochimique est compatible avec l'extension de filtre et/ou c) le masque respiratoire.

12. Procédé de surveillance in situ (de préférence portable) ou clinique de la présence d'un analyte dans l'air expiré d'un sujet comprenant

a. la fourniture d'un capteur électrochimique selon l'une quelconque des revendications 1 à 10 ou d'un système d'analyse et/ou de surveillance de l'air expiré selon la revendication 11 précédente
b. le positionnement dudit capteur électrochimique dans le flux respiratoire dudit sujet
c. l'utilisation d'une mesure différentielle en détectant le signal électrochimique différentiel au niveau de l'au moins une paire d'électrodes de travail afin de surveiller la présence de l'analyte.

13. Procédé selon la revendication précédente **caractérisé en ce que**

la présence de l'analyte est surveillée en continu pendant un seul ou de multiples cycles d'expiration et d'inspiration et/ou dans lequel différents segments du signal surveillé sont utilisés afin de quantifier la présence de l'analyte dans différentes régions d'un poumon et/ou des voies respiratoires.

14. Procédé selon l'une quelconque des revendications 12 ou 13 **caractérisé en ce que**

le signal détecté au niveau de l'électrode témoin insensible à l'analyte est utilisé pour une correction de fond des interférences non spécifiques du signal détecté au niveau de ladite électrode témoin sensible à l'analyte, dans lequel de préférence le procédé de correction de fond est capable de compenser les variations de courant causées par les mouvements respiratoires et les conditions environnementales.

**Fig. 1**

**Fig. 2**

Fig. 3

**Fig. 4**

**Fig. 5**

**Fig. 6**

Fig. 7

Fig. 8

Fig. 9

n = 7
y = 0.23x - 1.53
R² = 0.9887

Fig. 10

Fig. 11

Fig. 12

Fig. 13

Fig. 14

**Fig. 15**

**Fig. 16**

Fig. 17

Fig. 18

Fig. 19

Wires to reader

Conventional respiratory mask

Chip connector

Paper-based sensor isolated with flexible tape

Fig. 20

Conventional respiratory mask with integrated paper-based sensor

Wires to the potentiostat connected with a smartphone

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012067511 A1
- US 201300919224 A1
- WO 9844342 A

- WO 2013090999 A1
- US 4816567 A

### Non-patent literature cited in the description

- **KOHLER et al.** *Nature*, 1975, vol. 256, 495
- **CLACKSON et al.** *Nature*, 1991, vol. 352, 624-628
- **MARKS et al.** *J. Mol. Biol.*, 1991, vol. 222, 581-597
- **MORRISON et al.** *Proc. NatL. Acad Sci. USA*, 1984, vol. 81, 6851-6855
- **MARINESCU, M.** ; **WINTER, J.** Grundlagenwissen Elektrotechnik: Gleich-, Wechsel- und Drehstrom. Vieweg+Teubner Verlag, 2011
- **WORLD HEALTH ORGANIZATION.** *WHO releases country estimates on air pollution exposure and health impact*, 2016
- **WORLD HEALTH ORGANIZATION.** *The Global Impact of Respiratory Disease*, 2017
- **WORLD HEALTH ORGANIZATION.** *Chronic respiratory diseases*, 2004
- **WORLD HEALTH ORGANIZATION.** *Fact sheet - Noncommunicable diseases*, 23 August 2018, www.who.int/news-room/fact-sheets/detail/noncommunicable-diseases
- **ZHOU, M.** ; **LIU, Y.** ; **DUAN, Y.** Breath biomarkers in diagnosis of pulmonary diseases. *Clin. Chim. Acta*, 2012, vol. 413, 1770-1780
- **SVENSSON, S.** ; **OLIN, A.** ; **LARSTAD, M.** ; **LJUNGKVIST, G.** ; **TOREN, K**. Determination of hydrogen peroxide in exhaled breath condensate by flow injection analysis with fluorescence detection. *J. Chromatogr. B*, 2004, vol. 809, 199-203
- **PAGET-BROWN, A. O. et al.** Normative Data for pH of Exhaled Breath Condensate. *Chest*, 2006, vol. 129, 426-430
- **SOLVAY CHEMICALS INC**. *Hydrogen peroxide safety and handling, technical datasheet*, 2015, https://www.solvay.us/en/binaries/HH-2323-236798.pdf.
- **HORVÁTH, I.** ; **HUNT, J.** ; **BARNES, P. J**. Exhaled breath condensate: methodological recommendations and unresolved questions. *Eur. Respir. J.*, 2005, vol. 26, 523-548

- **GERRITSEN, W. B. M.** ; **ASIN, J.** ; **ZANEN, P.** ; **BOSCH, J. M. M. VA. DEN** ; **HAAS, F. J. L. M**. Markers of inflammation and oxidative stress in exacerbated chronic obstructive pulmonary disease patients. *Respir. Med.*, 2005, vol. 99, 84-90
- **DOHLMAN, A. W.** ; **BLACK, H. R.** ; **ROYALL, J. A**. Expired Breath Hydrogen Peroxide Is a Marker of Acute Airway Inflammation in Pediatric Patients with Asthma. *Am. Rev. Respir. Dis.*, 1993, vol. 148, 955-960
- **KOMKOVA, M. A.** ; **KARYAKINA, E. E.** ; **MARKEN, F.** ; **KARYAKIN, A. A**. Hydrogen Peroxide Detection in Wet Air with a Prussian Blue Based Solid Salt Bridged Three Electrode System. *Anal. Chem.*, 2013, vol. 85, 2574-2577
- **MUTLU, G. M.** ; **GAREY, K. W.** ; **ROBBINS, R. A.** ; **DANZIGER, L. H.** ; **RUBINSTEIN, I**. Collection and analysis of exhaled breath condensate in humans. *Am. J. Respir. Crit. Care Med.*, 2001, vol. 164, 731-7
- **DINCER, C. et al.** Disposable Sensors in Diagnostics, Food, and Environmental Monitoring. *Adv. Mater.*, 2019, 1806739
- **BANDODKAR, A. J.** ; **JEERAPAN, I.** ; **WANG, J**. Wearable Chemical Sensors: Present Challenges and Future Prospects. *ACS Sensors*, 2016, vol. 1, 464-482
- **KENRY, YEO, J. C.** ; **LIM, C. T**. Emerging flexible and wearable physical sensing platforms for healthcare and biomedical applications. *Microsystems Nanoeng*, 2016, vol. 2, 16043
- **KOYDEMIR, H. C.** ; **OZCAN, A**. Wearable and Implantable Sensors for Biomedical Applications. *Annu. Rev. Anal. Chem.*, 2018, 11
- **YANG, Y.** ; **GAO, W**. Wearable and flexible electronics for continuous molecular monitoring. *Chem. Soc. Rev.*, 2019, vol. 48, 1465-1491
- **ROLLAND, J. P.** ; **MOUREY, D. A**. Paper as a novel material platform for devices. *MRS Bull*, 2013, vol. 38, 299-305

- **DINCER, C.** ; **BRUCH, R.** ; **KLING, A.** ; **DITTRICH, P. S.** ; **URBAN, G. A**. Multiplexed Point-of-Care Testing - xPOCT. *Trends Biotechnol.*, 2017, vol. 35, 728-742
- **BARANDUN, G. et al.** Cellulose fibers enable near zero-cost electrical sensing of water-soluble gases. *ACS Sensors*, 2019
- **BRACHER, P. J.** ; **GUPTA, M.** ; **WHITESIDES, G. M.** Patterning precipitates of reactions in paper. *J. Mater. Chem.*, 2010, vol. 20, 5117
- **GÜDER, F. et al.** Paper-Based Electrical Respiration Sensor. *Angew. Chemie*, 2016, vol. 55, 5727-5732
- **GASPAR, C.** ; **OLKKONEN, J.** ; **PASSOJA, S.** ; **SMOLANDER, M**. Paper as Active Layer in Inkjet-Printed Capacitive Humidity Sensors. *Sensors*, 2017, vol. 17, 1464
- **GRELL, M. et al.** Autocatalytic Metallization of Fabrics Using Si Ink, for Biosensors, Batteries and Energy Harvesting. *Adv. Funct. Mater.*, 2019, vol. 29, 1804798
- **WHO**. *Diabetes*, 2020, https://www.who.int/health-topics/diabetes
- Recent developments in minimally and truly non-invasive blood glucose monitoring techniques. **CHOI, H**. 2017 IEEE SENSORS. IEEE, 2017, 1-3
- **DINCER, C. et al.** Disposable Sensors in Diagnostics, Food, and Environmental Monitoring. *Adv. Mater.*, 2019, vol. 31, 1806739
- **KOVALASKE, M. A.** ; **GANDHI, G. Y**. Glycemic Control in the Medical Intensive Care Unit. *J. Diabetes Sci. Technol.*, 2009, vol. 3, 1330-1341
- **TANKASALA, D.** ; **LINNES, J. C**. Noninvasive glucose detection in exhaled breath condensate. *Transl. Res.*, 2019, vol. 213, 1-22
- **FERRANTE DO AMARAL, C. E.** ; **WOLF, B**. Current development in non-invasive glucose monitoring. *Med. Eng. Phys.*, 2008, vol. 30, 541-549
- **NERY, E. W.** ; **KUBOTA, L. T**. Evaluation of enzyme immobilization methods for paper-based devices-A glucose oxidase study. *J. Pharm. Biomed. Anal.*, 2016, vol. 117, 551-559
- **GHORESIHIZADEH SARA S**. An integrated Platform for diffrential electrochemical and ISFET sensing. *IEEE International Symposium on circuits and systems (ISCAS)*, 2016
- **GÜDER FIRAT et al.** Paper-Based Electrical Respiration Sensor. *Angewandte Chemie*, 2016, vol. 55, 5727-5732